(19) Europäisches Patentamt
European Patent Office
Office européen des brevets

(11) **EP 3 479 263 B1**

(12) **EUROPEAN PATENT SPECIFICATION**

(45) Date of publication and mention of the grant of the patent:
**10.08.2022 Bulletin 2022/32**

(21) Application number: **17732416.7**

(22) Date of filing: **22.06.2017**

(51) International Patent Classification (IPC):
*G16H 20/17* (2018.01)    *A61B 5/00* (2006.01)
*A61B 5/145* (2006.01)    *G16H 50/20* (2018.01)
*G16H 40/67* (2018.01)    *G16H 50/50* (2018.01)

(52) Cooperative Patent Classification (CPC):
**A61B 5/0022; A61B 5/14532; G16H 20/17; G16H 40/67; G16H 50/20; G16H 50/50**

(86) International application number:
**PCT/EP2017/065385**

(87) International publication number:
**WO 2018/001855 (04.01.2018 Gazette 2018/01)**

(54) **SYSTEMS AND METHODS FOR ANALYSIS OF INSULIN REGIMEN ADHERENCE DATA**

SYSTEME UND VERFAHREN ZUR TEMPORALER PERIODIZITÄTSANALYSE VON INSULINDIÄT-EINHALTUNGSDATEN

SYSTÈMES ET PROCÉDÉS POUR L'ANALYSE DE LA PÉRIODICITÉ TEMPORELLE DE DONNÉES D'OBSERVANCE DE TRAITEMENT POSOLOGIQUE PAR INSULINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **30.06.2016 EP 16177083**

(43) Date of publication of application:
**08.05.2019 Bulletin 2019/19**

(73) Proprietor: **Novo Nordisk A/S 2880 Bagsværd (DK)**

(72) Inventors:
• **BENGTSSON, Henrik**
  **2880 Bagsværd (DK)**
• **ARADÓTTIR, Tinna, Björk**
  **2880 Bagsværd (DK)**
• **BROCKMEIER, Pete**
  **2880 Bagsværd (DK)**

(56) References cited:
WO-A2-2006/072416    WO-A2-2010/149388
US-A1- 2007 179 352    US-A1- 2014 019 396
US-A1- 2015 006 462

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## TECHNICAL FIELD

The present disclosure relates generally to systems and methods for assisting patients and health care practitioners in identifying periodic nonadherence to prescribed insulin medicament dosage regimens as a basis for determining what improvements to regimen adherence will favorably affect glucose levels.

## BACKGROUND

Type 2 diabetes mellitus is characterized by progressive disruption of normal physiologic insulin secretion. In healthy individuals, basal insulin secretion by pancreatic β cells occurs continuously to maintain steady glucose levels for extended periods between meals. Also in healthy individuals, there is prandial secretion in which insulin is rapidly released in an initial first-phase spike in response to a meal, followed by prolonged insulin secretion that returns to basal levels after 2-3 hours.

Insulin is a hormone that binds to insulin receptors to lower blood glucose by facilitating cellular uptake of glucose, amino acids, and fatty acids into skeletal muscle and fat and by inhibiting the output of glucose from the liver. In normal healthy individuals, physiologic basal and prandial insulin secretions maintain euglycemia, which affects fasting plasma glucose and postprandial plasma glucose concentrations. Basal and prandial insulin secretion is impaired in Type 2 diabetes and early post-meal response is absent. To address these adverse events, patients with Type 2 diabetes are provided with insulin treatment regimens. Patients with Type 1 diabetes are also provided with insulin treatment regimens.

Some diabetic patients only need a basal insulin treatment regimen to make up for deficiencies in pancreatic β cells insulin secretion. Some patients need both basal insulin treatment and bolus insulin treatment. Thus, patients that require both basal insulin treatment and bolus insulin treatment take a periodic basal insulin medicament treatment, for instance once or twice a day, as well as one or more bolus insulin medicament treatments with meals.

The goal of these insulin treatment regimens is to achieve steady glucose levels. The success of an insulin treatment regimen in a subject can be deduced by taking continuous glucose level measurements of a subject or by measuring HbAlc levels. The term "HbA1c" refers to glycated haemoglobin. It develops when haemoglobin, a protein within red blood cells that carries oxygen throughout the body, joins with glucose in the blood, thus becoming "glycated." By measuring glycated haemoglobin (HbA1c), health care practitioners are able to get an overall picture of average glucose levels over a period of weeks/months. For people with diabetes, the higher the HbA1c, the greater the risk of developing diabetes-related complications

Insulin treatment regimen nonadherence is a barrier for diabetes patients to reaching suitable HbAlc goals. Insulin regimen adherence is typically defined as the degree to which a patient correctly follows medical advice (e.g., a standing insulin regimen for a subject comprising at least a basal insulin medicament dosage regimen), but can also be, for example, consistency in diet and exercise. The reasons for nonadherence are many and different. One reason for nonadherence is poor health literacy and comprehension of treatment. Patients fail to understand glucose measurement results, lack positive feedback when adherent, or feel a lack of urgency. Another reason for nonadherence is the fear of side effects. For instance, the fear of hypoglycaemia if the patient strictly adheres to the standing insulin regimen. Yet another reason for nonadherence is the hassle and time-consuming aspect of conventional standing insulin regimens, which often entail home-logging data and frequent injections and glucose measurements. Still another reason for nonadherence is an inability to pinpoint the source of nonadherence that is the actual source of the adverse effect on stable glucose levels.

International Publication Number WO 2012/152295 A2 to Insulin Medical Ltd. optimizes insulin absorption by using one or more sensors and actuators configured to provide data relating to a user's meal status, meal timing, the timing of administered drug, drug dose, drug type, the logging of user activity, and the analysis thereof. For instance, WO 2012/152295 A2 discloses a device that may be placed over an injection site or an injection port to treat the tissue at the injection site, while collecting information on the injected drug at the time of injections with an option to provide feedback to the user, such as alerts on missed injections. WO 2012/152295 A2 further discloses using meal data and other subject data, such as the activity of the subject, to facilitate mapping the subject activity relative to injection events and optionally meal events to provide for fine control of the systemic metabolic process of glucose and insulin and therefore minimize occurrence of post prandial hyperglycemic and hypoglycemic events. However, WO 2012/152295 A2 fails to provide satisfactory ways to determine and quantify the effects of insulin regimen adherence, or lack thereof, on the health of a subject (e.g., glucose levels of the subject) or to provide guidance on what forms of insulin regimen adherence would benefit a subject. In essence, WO 2012/152295 A2 fails to provide satisfactory ways to pinpoint precisely what forms of regimen nonadherence are most adversely affecting glucose levels. Moreover, generally, WO 2012/152295 A2 fails to provide overall feedback on the subject's adherence to an insulin medicament regimen. Further, the meal detection in WO 2012/152295 A2 is not based upon autonomous glucose measurements and thus the reliability of the meal detection in WO 2012/152295 A2 is uncertain.

International Publication Number WO 2014/037365 A1 to Roche Diagnostics GMBH describes methods and apparatuses for analyzing blood glucose data and events, and, in particular, to computer implemented methods for visualizing correlations between blood glucose data and events associated with the blood glucose data such as meals. However, WO 2014/037365 A1 fails to disclose any categorization of meals in terms of insulin regimen adherence. Further, WO 2014/037365 A1 fails to provide satisfactory ways in which to determine and quantify the effects of insulin regimen adherence, or lack thereof, on the health of a subject or to provide guidance on what forms of insulin regimen adherence would benefit a subject.

International Publication Number WO 2010/149388 A2 to Roche Diagnostics GMBH, upon which the two-part form of claim 1 is based, describes a method of measuring adherence to following or achieving prescribed therapy steps to achieve stated target goals for improved chronic disease self-management. The method comprises defining a plurality of adherence units, each adherence unit containing a plurality of rules governing activities which need to be accomplished in order to complete the prescribed therapy steps; collecting data when the activities are accomplished specifying a time window of interest in the collected data; determining total number of adherence units in the collected data which fall within the specified time window of interest; counting each of the adherence units in the specified time window of interest as an adhered unit when the collected data indicates the accomplished activities were in accordance to the rules; determining adherence as a percentage of the count for the adhered units to the total number of adherence units for the specified time window; and providing at least one of the determined adherence percentage and adherence count for the specified time window. The publication further describes that a time period is the start and end of time describing the absolute time window during which all the recorded activities are considered, and that a subset time period is the subset of a time window within the time period. The subset time period covers event with certain periodicity. For example, a subset time period can be a breakfast activity covering Mondays only. The publication further describes, a computer program, when running on a processing device, instructing the processing device to collect the data regarding an individual's activities per the prescribed (i.e., inputted and selected) protocol(s). The information regarding each activity is captured by the processing device by the computer program instructing the processing device to prompt the individual via the user interface or other suitable output hardware and to accept user inputs providing the information. The computer program then stores the inputted information in a memory of the processing device as collected data. In one embodiment, the computer program annotates the collected data regarding the protocol and/or activity, such as with a timestamp of start and completion, contextual information, and other relevant quantified and subjective data. Recording of the activity and managing the associated information via the above-mentioned data collection processes enable such data to be analyzed in order to provide an assessment of an individual's adherence level. In particular, via the data collection processes, the data information and associations are captured within the memory of the processing device (or a database) such that the recorded sequence of activities has no ambiguity. The collected data is then utilized in later steps for extracting relevant subsets of data, applying adherence rules, and providing a number either as a ratio or in percentage format or an equivalent which indicates the extent to which adherence is accomplished. Even though an activity unit is generally of finite duration, the start of activity is considered as the absolute time for the activity unit 16. For example, a breakfast activity time is the time at which the breakfast activity unit is initiated. If the breakfast activity consists of a number of activity steps, such as for example, estimating carbohydrates in the breakfast meal, followed by measuring blood glucose (BG), followed by computation of insulin dose, followed by eating of the breakfast meal, followed by a 2-hour post-prandial measuring of BG, then the breakfast activity is timed as per preference or choice for marking the activity as preferably suggested by physician, so for example when the individual starts the estimation of the carbohydrate in the breakfast. As appears, WO 2010/149388 A2 relies on collecting data by prompting a user, and the collected data, therefore, comprises user input activities. WO 2010/149388 does not solve the problem of measuring adherence of a metabolic activity relevant to the prescribed regimen, in situations where a user forgets to input when prompted, is unable to answer when prompted or for some reason inputs wrong data to the memory when prompted, and it does not solve the problem of directly monitoring adherence based on a metabolic activity that a user has engaged in, and not merely intends to engage in, or have engaged in a while ago. Furthermore periods of low adherence may be associated with the subject being less reliable in inputting when prompted, i.e., weekends where the subject is engaged in certain social activities. In other words the timing between user input activities and the metabolic activity relevant for monitoring adherence of a prescribed regimen is subject to uncertainty.

Given the above background, what is needed in the art are systems and methods that provide satisfactory ways to pinpoint what forms of regimen nonadherence are adversely affecting glucose levels in diabetic patients.

The object of the present disclosure is to provide systems and methods for reliably monitoring and communicating insulin regimen adherence and to pinpoint what forms of regimen nonadherence are adversely affecting glucose levels in diabetic patients.

## SUMMARY

In the disclosure of the present invention, embodiments and aspects will be described, which will address one or more of the above objects or which will address objects apparent from the below disclosure as well as from the description of exemplary embodiments.

The present disclosure addresses the above-identified need in the art by providing methods and apparatus for assisting patients and health care practitioners in identifying periodic nonadherence to prescribed insulin medicament dosage regimens as a basis for determining what improvements to regimen adherence will favorably affect glucose levels.

Using the systems and method of the present disclosure, patients or health care practitioners can determine what form of regimen nonadherence most adversely affects glucose levels. For instance, using the systems and methods of the present disclosure, periodic patterns of noncompliance can be elucidated, as well as their effect on stable glucose levels.

In one aspect of the present disclosure, systems and methods are provided for monitoring adherence to a prescribed insulin medicament dosage regimen for a subject over time. A first data set is obtained at a device. The first data set comprises a plurality of metabolic events in which the subject engaged. Each respective metabolic event in the plurality of metabolic events comprises (i) a timestamp of the respective metabolic event and (ii) a first classification that is one of insulin regimen adherent and insulin regimen nonadherent.

Each respective metabolic event in the plurality of metabolic events is classified using a second classification based upon the timestamp of the respective metabolic event. The second classification is characterized by a temporal periodicity and includes a plurality of periodic elements. Once classified according to the second classification, each respective metabolic event in the plurality of metabolic events is binned on the basis of the second classification thereby obtaining a plurality of subsets of the plurality of metabolic events. Each respective subset of the plurality of metabolic events in the plurality of subsets is for a different periodic element in the plurality of periodic elements.

For each respective subset in the plurality of subsets, there is communicated a respective representation of adherence to the prescribed insulin medicament dosage regimen. The respective representation of adherence for a given subset is collectively based upon the first classification of metabolic events in the respective subset. In this way adherence to the prescribed insulin medicament dosage regimen for the subject over time is monitored.

The method comprises the further steps of: obtaining a second data set comprising a plurality of autonomous glucose measurements of the subject and, for each respective autonomous glucose measurement in the plurality of autonomous glucose measurements, a timestamp representing when the respective measurement was made, obtaining a third data set from one or more insulin pens used by the subject to apply the insulin medicament dosage regimen, the third data set comprises a plurality of insulin medicament records, each insulin medicament record in the plurality of medicament records comprising: (i) a respective insulin medicament injection event including an amount of insulin medicament injected into the subject using a respective insulin pen in the one or more insulin pens and (ii) a corresponding electronic timestamp that is automatically generated by the respective insulin pen upon occurrence of the respective insulin medicament injection event, identifying the plurality of metabolic events using the plurality of autonomous glucose measurements of the subject and the respective timestamps in the second data set, and applying a first characterization to each respective metabolic event in the plurality of metabolic events, wherein the first characterization is one of insulin regimen adherent and insulin regimen nonadherent.

A respective metabolic event is deemed basal regimen adherent when the second data set includes one or more medicament records that establish, on a temporal and quantitative basis, adherence with the insulin medicament dosage regimen during the respective metabolic event, and a respective metabolic event is deemed insulin regimen nonadherent when the second data set fails to include one or more medicament records that establish, on a temporal and quantitative basis, adherence with the insulin medicament dosage regimen,

Alternatively, the plurality of metabolic events are meal events, a respective meal event is deemed insulin regimen adherent when one or more medicament records in the plurality of medicament records indicates in the third data set, on a temporal basis, a quantitative basis, adherence with the insulin medicament dosage regimen during the respective meal, and a respective meal is deemed insulin regimen nonadherent when the plurality of medicament records in the third data set fails to indicate adherence, on a temporal basis, and a quantitative basis with the insulin medicament dosage regimen during the respective meal. Hereby is provided a system and method which establishes adherence monitoring based on metabolic events, which the subject actually engaged in, and thereby eliminates the risk of user behavior not always follows expectations.

The system and the method solve the problem of how to systematically allow tracking of periodic adherence or nonadherence based on well-defined and reliable reference points in time. As the data set only comprises metabolic events that the subject engaged in, the system and the method does not rely on input on a user response, and it thereby solves the problem of prior art. As the data set comprises timestamps for each metabolic event, which the subject engaged in the adherence is monitored with a high degree of uncertainty. The use of data comprising metabolic events that the subject actually engaged in for the purpose of monitoring adherence has not been previously used or described, nor has the importance of using such data in order to minimize uncertainty of the monitored adherence.

In a further aspect, autonomous measurements are measurements obtained by a measuring device, wherein the measuring is undertaken or carried on without outside control of a user. Hereby is provided data that do not rely on input controlled by the subject or an operator of the device.

In a further aspect, autonomous measurements are measurements obtained by a device measuring at a specified or a variable frequency.

In some embodiments, each respective metabolic event in the plurality of metabolic events is within a period of time that spans a plurality of weeks, the temporal periodicity is weekly, and each periodic element in the plurality of metabolic events is a different day in the seven days of the week. In some such embodiments, each respective metabolic event in the plurality of metabolic events is a fasting event and the insulin medicament dosage regimen is a basal insulin medicament dosage regimen.

In other embodiments, each respective metabolic event in the plurality of metabolic events is within a period of time that spans a plurality of days, each respective metabolic event in the plurality of metabolic events is a meal event, and the insulin medicament dosage regimen is a bolus insulin medicament dosage regimen. In some such embodiments, the temporal periodicity is daily, and each periodic element in the plurality of periodic elements is a different one of "breakfast," "lunch," and "dinner." In other embodiments, the temporal periodicity is weekly, and each periodic element in the plurality of periodic elements represents a different meal in a set of 21 calendared weekly meals.

In some embodiments, the respective representation of adherence for each respective subset in the plurality of subsets is collectively represented as a continuous two-dimensional spiral timeline comprising a plurality of revolutions. This spiral timeline comprises a plurality of radial sectors, and each revolution in the plurality of revolutions represents a period of the temporal periodicity. Further, each respective radial sector in the plurality of radial sectors is uniquely assigned a corresponding subset in the plurality of subsets.

In some embodiments each respective adherence value in the plurality of adherence values represents a corresponding time window in a plurality of time windows. Further, each respective time window in the plurality of time windows is of a same first fixed duration. In such embodiments, each respective adherence value in the plurality of adherence values is computed by dividing a number of insulin regimen adherent metabolic events by a total number of metabolic events in the plurality of metabolic events that have timestamps in the time window corresponding to the respective adherence value. Further, each respective adherence value in the plurality of adherence values is assigned to a respective radial sector in the plurality of radial sectors based upon a time period represented by the respective adherence value thereby forming, for each respective subset in the plurality of subsets, the respective representation of adherence with the prescribed insulin medicament dosage regimen. In some such embodiments, each respective adherence value in the two-dimensional spiral timeline is color coded as a function of an absolute value of the respective adherence value. In some such embodiments, the continuous two-dimensional spiral is an Archimedean spiral or a logarithmic spiral.

In some embodiments, the device used to perform any one of the above identified methods includes a display and that presents each respective representation of adherence with the prescribed insulin medicament dosage regimen on the display. In some such embodiments, the device is a mobile device.

In some embodiments, each respective autonomous glucose measurement in the plurality of autonomous glucose measurements is classified using the second classification, based upon the timestamp of the respective autonomous glucose measurement. Further, each respective subset in the plurality of subsets is communicated with those values of autonomous glucose measurements in the plurality of autonomous glucose measurements that have been classified into the same periodic element in the plurality of periodic elements that the respective subset represents. In some such embodiments, the device further comprising a wireless receiver, and the second data set is obtained wirelessly from a glucose sensor affixed to the subject.

In a further aspect the medicament record further comprises a type of insulin medicament, and wherein, a respective fasting event is deemed insulin regimen adherent when one or more medicament records in the plurality of medicament records further indicates in the third data set, on a type of insulin medicament basis, adherence with the standing insulin medicament dosage regimen during the respective fasting event, and a respective fasting event is deemed insulin regimen nonadherent when the plurality of medicament records in the third data set further fails to indicate adherence, on a type of insulin medicament basis with the insulin medicament dosage regimen during the respective fasting period.

In a further aspect the medicament record further comprises a type of insulin medicament, and wherein, a respective meal event is deemed insulin regimen adherent when one or more medicament records in the plurality of medicament records further indicates in the third data set, on a type of insulin medicament basis, adherence with the insulin medicament dosage regimen during the respective meal, and a respective meal is deemed insulin regimen nonadherent when the plurality of medicament records in the third data set further fails to indicate adherence, on a type of insulin medicament basis with the insulin medicament dosage regimen during the respective meal.

In a further aspect the insulin regimen adherent is defined as bolus regimen adherent, and insulin regiment nonadherent is defined as bolus regimen nonadherent.

In a further aspect, the metabolic events are inherently timestamped, i.e., the timestamp of the metabolic event is a direct consequence of the occurrence of the metabolic event and the timestamp is acquired in response to this occurrence.

Hereby is provided a system ensuring that adherence is monitored with respect to metabolic events that the subject has engaged in, and as the metabolic event is timestamped there is provided a well-defined reference in time, allowing the classification of adherence to utilize the timestamp.

In a further aspect, the timestamp relating to a respective metabolic event is used as a starting point for determining whether the metabolic event is insulin regimen adherent or insulin regimen nonadherent.

A metabolic event defined according to the medicament regimen could be a meal event, wherein the medicament regimen determines that bolus insulin should be administered based on glucose measurements relating to this event, or it could be a fasting event, wherein the medicament regimen determines that basal insulin should be administered based on glucose measurements relating to this event.

In a further aspect the method further comprises computing a plurality of primary adherence values, wherein each respective primary adherence value in the plurality of primary adherence values represents a corresponding periodic element in the plurality of periodic elements, and each respective primary adherence value in the plurality of primary adherence values is computed by dividing a number of insulin regimen adherent metabolic events in each respective subset by a total number of metabolic events in the respective subset corresponding to the respective periodic element, and wherein the respective representation of adherence for each respective subset in the plurality of subsets is collectively represented as the corresponding primary adherence value.

Another aspect of the present disclosure provides a method of monitoring adherence to a prescribed insulin regimen for a subject as defined in claim 15.

**BRIEF DESCRIPTION OF THE DRAWINGS**

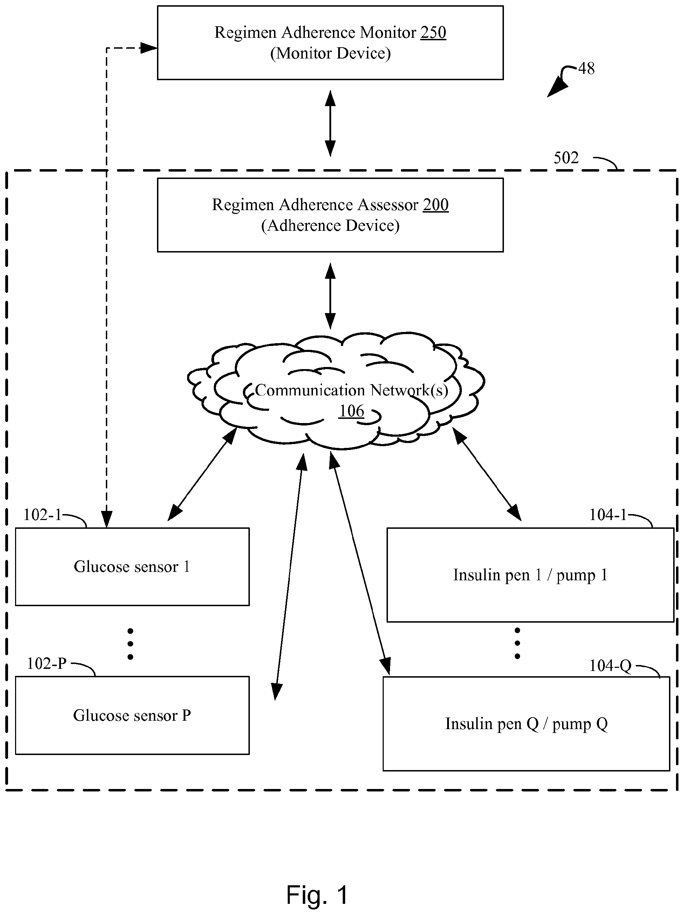

Figure 1 illustrates an exemplary system topology that includes a regimen adherence monitor device for monitoring adherence to a prescribed insulin medicament dosage regimen for a subject over time, a regimen adherence assessor device for analyzing and preparing regimen adherence data, one or more glucose sensors that measure glucose data from the subject, and one or more insulin pens or pumps that are used by the subject to inject insulin medicaments in accordance with the prescribed insulin medicament dosage regimen, where the above-identified components are interconnected, optionally through a communications network, in accordance with an embodiment of the present disclosure.

Figure 2 illustrates a device for monitoring adherence to a prescribed insulin medicament dosage regimen for a subject over time in accordance with an embodiment of the present disclosure.

Figure 3 illustrates a device for monitoring adherence to a prescribed insulin medicament dosage regimen for a subject over time in accordance with another embodiment of the present disclosure.

Figures 4A, 4B, and 4C collectively provide a flow chart of processes and features of a device for monitoring adherence to a prescribed insulin medicament dosage regimen for a subject over time in accordance with various embodiments of the present disclosure.

Figure 5 illustrates an example integrated system of connected insulin pen(s), continuous glucose monitor(s), memory and a processor for performing algorithmic categorization of autonomous glucose data in accordance with an embodiment of the present disclosure.

Figure 6 illustrates an algorithm for classifying metabolic events in accordance with an embodiment of the present disclosure.

Figure 7 illustrates the classification of each respective metabolic event in a plurality of metabolic events, using a second classification, based upon a timestamp of the respective metabolic event, where the second classification is characterized by a temporal periodicity and includes a plurality of periodic elements, in accordance with an embodiment of the present disclosure.

Figure 8 illustrates the classification of each respective metabolic event in a plurality of metabolic events, using a second classification, based upon a timestamp of the respective metabolic event, where the second classification is characterized by a temporal periodicity and includes the periodic elements "Breakfast," "Lunch," and "Dinner," in accordance with an embodiment of the present disclosure.

Figure 9 illustrates binning each respective metabolic event in a plurality of metabolic events on the basis of a second classification thereby obtaining a plurality of subsets of the plurality of metabolic events in accordance with one embodiment of the present disclosure.

Figure 10 illustrates binning each respective metabolic event in a plurality of metabolic events on the basis of a second classification thereby obtaining a plurality of subsets of the plurality of metabolic events in accordance with another embodiment of the present disclosure.

Figure 11 illustrates the respective representation of adherence for each respective subset in a plurality of subsets collectively represented as a continuous two-dimensional spiral timeline comprising a plurality of revolutions in accordance with an embodiment of the present disclosure.

Figure 12 illustrates the computation of adherence values from the first classification of metabolic events for periodic elements in subsets in accordance with an aspect of the present disclosure.

Like reference numerals refer to corresponding parts throughout the several views of the drawings.

## DETAILED DESCRIPTION

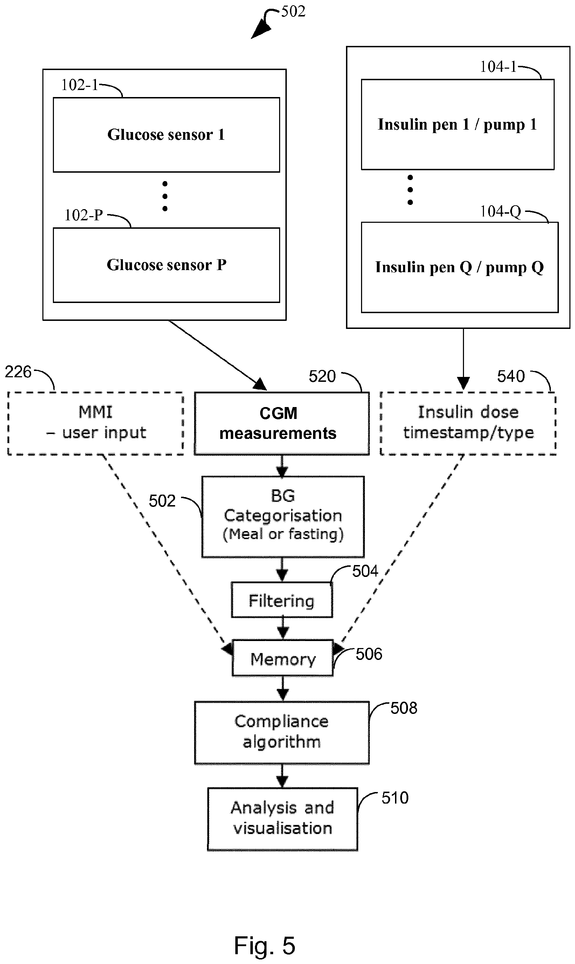

The present disclosure relies upon the acquisition of data regarding a plurality of metabolic events, such as fasting events or meals, a subject engaged in over a period of time. For each such metabolic event, the data includes a timestamp and a classification of the metabolic event that is either insulin regimen adherent or insulin regimen nonadherent. Figure 1 illustrates an example of an integrated system 502 for the acquisition of such data, and Figure 5 provides more details of such a system 502. The integrated system 502 includes one or more connected insulin pens or pumps 104, one or more continuous glucose monitors 102, memory 506, and a processor (not shown) for performing algorithmic categorization of autonomous glucose data of a subject.

A metabolic event is an event relating to metabolism, which is the sum of the processes in the buildup and destruction of protoplasm, e.g., the chemical changes in living cells by which energy is provided for vital processes and activities and new material is assimilated, i.e., utilized as nourishment.

The metabolism in a living body can be defined in different states: an absorptive state, or fed state, occurs after a meal when the body is digesting food and absorbing nutrients. Digestion begins the moment food enters the mouth, as the food is broken down into its constituent parts to be absorbed through the intestine. The digestion of carbohydrates begins in the mouth, whereas the digestion of proteins and fats begins in the stomach and small intestine. The constituent parts of these carbohydrates, fats, and proteins are transported across the intestinal wall and enter the bloodstream (sugars and amino acids) or the lymphatic system (fats). From the intestines, these systems transport them to the liver, adipose tissue, or muscle cells that will process and use, or store, the energy. In the absorptive state glucose, lipids and amino acids enter the blood stream and insulin may be released (depending on the other conditions like the state and type of diabetes). The postabsorptive state, or the fasting state, occurs when the food has been digested, absorbed, and stored. You commonly fast overnight, but skipping meals during the day puts your body in the postabsorptive state as well. During this state, the body must rely initially on stored glycogen. Glucose levels in the blood begin to drop as it is absorbed and used by the cells. In response to the decrease in glucose, insulin levels also drop. Glycogen and triglyceride storage slows. However, due to the demands of the tissues and organs, blood glucose levels must be maintained in the normal range of 80-120 mg/dL. In response to a drop in blood glucose concentration, the hormone glucagon is released from the alpha cells of the pancreas. Glucagon acts upon the liver cells, where it inhibits the synthesis of glycogen and stimulates the breakdown of stored glycogen back into glucose. This glucose is released from the liver to be used by the peripheral tissues and the brain. As a result, blood glucose levels begin to rise. Gluconeogenesis will also begin in the liver to replace the glucose that has been used by the peripheral tissues. Further information can be found in OpenStax College, Anatomy and Physiology. OpenStax CNX. http://cnx.org/contents/14fb4ad7-39a1-4eee-ab6e-3ef2482e3e22@8.81.

A metabolic event may therefore relate to an event where a certain metabolic state occurs, and the occurrence may be detected by measuring the concentration of an indicator of the event. The metabolic event will be an indicator of the type of state, and the progress of the state, and an indicator of a metabolic event can be the concentration of glucose, glucagon, lipids and amino acids in the blood stream. Other hormones may also be useful for determining events relating to metabolism like cortisol and adrenaline.

Autonomous measurements or autonomous data are measurements or data obtained by a device measuring at a specified or a variable frequency, wherein the measuring is undertaken or carried on without outside control, e.g., when the device is operating in a measurement mode the measuring can be performed without control from the a subject using the device.

Referring to Figure 5, with the integrated system 502, autonomous timestamped glucose measurements of the subject are obtained 520. Also, data from the one or more insulin pens and/or pumps used to apply a prescribed insulin regimen to the subject is obtained 540 as a plurality of records. Each record comprises a timestamped event specifying an amount of injected (or pumped) insulin medicament that the subject received as part of the prescribed insulin medicament dosage regimen. Fasting events are identified using the autonomous timestamped glucose measurements of the subject. Meal events are also identified using the autonomous timestamped glucose measurements 502. In this way, the glucose measurements are filtered 504 and stored in non-transitory memory 506.

A metabolic event is characterized as adherent or nonadherent. A metabolic event is adherent when one or more records from the one or more connected insulin pens or pumps 104 temporally and quantitatively establish adherence with the prescribed insulin medicament regimen. Conversely, a metabolic event is characterized as nonadherent when none of the records from the one or more connected insulin pens or pumps 104 temporally and quantitatively establish adherence with the prescribed basal insulin medicament regimen.

Each fasting event is classified as adherent or nonadherent 508. A fasting event is adherent when one or more records from the one or more connected insulin pens or pumps 104 temporally and quantitatively establish adherence with the prescribed basal insulin medicament regimen during the fasting event. Conversely, a fasting event is classified as nonadherent when none of the records from the one or more connected insulin pens or pumps 104 temporally and quantitatively establish adherence with the prescribed basal insulin medicament regimen.

A respective meal is deemed bolus regimen adherent when one or more medicament records indicates, on a temporal basis, a quantitative basis, and a type of insulin medicament basis, adherence with a prescribed bolus insulin medicament dosage regimen during the respective meal. Conversely, a respective meal is deemed bolus regimen nonadherent when the plurality of medicament records fails to indicate adherence, on a temporal basis, a quantitative basis, and a type of insulin medicament basis, with the prescribed bolus insulin medicament dosage regimen during the respective meal.

This filtered and cataloged glucose data is analyzed and visualized in accordance with the methods of the present disclosure 510. Such visualization enables the subject or health care practitioner to identify temporal insulin regimen adherence patterns and their effect on important subject biomarkers such as blood glucose levels and HbAlc levels.

Reference will now be made in detail to embodiments, examples of which are illustrated in the accompanying drawings. In the following detailed description, numerous specific details are set forth in order to provide a thorough understanding of the present disclosure. However, it will be apparent to one of ordinary skill in the art that the present disclosure may be practiced without these specific details. In other instances, well-known methods, procedures, components, circuits, and networks have not been described in detail so as not to unnecessarily obscure aspects of the embodiments.

It will also be understood that, although the terms first, second, etc. may be used herein to describe various elements, these elements should not be limited by these terms. These terms are only used to distinguish one element from another. For example, a first subject could be termed a second subject, and, similarly, a second subject could be termed a first subject, without departing from the scope of the present disclosure. The first subject and the second subject are both subjects, but they are not the same subject. Furthermore, the terms "subject" and "user" are used interchangeably herein. By the term insulin pen is meant an injection device suitable for applying discrete doses of insulin, and wherein the injection device is adapted for logging and communicating dose related data.

The terminology used in the present disclosure is for the purpose of describing particular embodiments only.

It will also be understood that the term "and/or" as used herein refers to and encompasses any and all possible combinations of one or more of the associated listed items. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

As used herein, the term "if" may be construed to mean "when" or "upon" or "in response to determining" or "in response to detecting," depending on the context. Similarly, the phrase "if it is determined" or "if [a stated condition or event] is detected" may be construed to mean "upon determining" or "in response to determining" or "upon detecting [the stated condition or event]" or "in response to detecting [the stated condition or event]," depending on the context.

A detailed description of a system 48 for monitoring adherence to a prescribed insulin medicament dosage regimen 206 for a subject over time in accordance with the present disclosure is described in conjunction with Figures 1 through 3. As such, Figures 1 through 3 collectively illustrate the topology of the system in accordance with the present disclosure. In the topology, there is a device for monitoring adherence to a prescribed insulin medicament dosage regimen ("monitor device 250") (Figures 1, 2, and 3), a device for assessing regimen adherence ("adherence device 200"), one or more glucose sensors 102 associated with the subject (Figure 1), and one or more insulin pens or pumps 104 for injecting insulin medicaments into the subject (Figure 1). Throughout the present disclosure, the adherence device 200 and the monitor device 250 will be referenced as separate devices solely for purposes of clarity. That is, the disclosed functionality of the adherence device 200 and the disclosed functionality of the monitor device 250 are contained in separate devices as illustrated in Figure 1. However, it will be appreciated that, in fact, in some embodiments, the disclosed functionality of the adherence device 200 and the disclosed functionality of the monitor device 250 are contained in a single device.

Referring to Figure 1, the monitor device 250 monitors adherence to an insulin medicament dosage regimen prescribed to a subject. To do this, the adherence device 200, which is in electrical communication with the monitor device 250, receives autonomous glucose measurements originating from one or more glucose sensors 102 attached to a subject on an ongoing basis. Further, the adherence device 200 receives insulin medicament injection data from one or more insulin pens and/or pumps 104 used by the subject to inject insulin medicaments. In some embodiments, the adherence device 200 receives such data directly from the glucose sensor(s) 102 and insulin pens and/or pumps 104 used by the subject. For instance, in some embodiments the adherence device 200 receives this data wirelessly through radio-frequency signals. In some embodiments such signals are in accordance with an 802.11 (WiFi), Bluetooth, or ZigBee standard. In some embodiments, the adherence device 200 receives such data directly, characterizes or classifies metabolic events within the data as regimen adherent or regimen nonadherent, and passes the classified data to the monitor device 250. In some embodiments the glucose sensor 102 and/or insulin pen / pump includes and RFID tag and communicates to adherence device 200 and/or the monitor device 250 using RFID communication.

In some embodiments, the adherence device 200 and/or the monitor device 250 is not proximate to the subject and/or does not have wireless capabilities or such wireless capabilities are not used for the purpose of acquiring glucose data and insulin medicament injection data. In such embodiments, a communication network 106 may be used to communicate glucose measurements from the glucose sensor 102 to the adherence device 200 and from the one or more insulin pens or pumps 104 to the adherence device 200.

Examples of networks 106 include, but are not limited to, the World Wide Web (WWW), an intranet and/or a wireless network, such as a cellular telephone network, a wireless local area network (LAN) and/or a metropolitan area network (MAN), and other devices by wireless communication. The wireless communication optionally uses any of a plurality of communications standards, protocols and technologies, including but not limited to Global System for Mobile Communications (GSM), Enhanced Data GSM Environment (EDGE), high-speed downlink packet access (HSDPA), high-speed uplink packet access (HSUPA), Evolution, Data-Only (EV-DO), HSPA, HSPA+, Dual-Cell HSPA (DC-HSPDA), long term evolution (LTE), near field communication (NFC), wideband code division multiple access (W-CDMA), code division multiple access (CDMA), time division multiple access (TDMA), Bluetooth, Wireless Fidelity (Wi-Fi) (e.g., IEEE 802.11a, IEEE 802.11ac, IEEE 802.11ax, IEEE 802.11b, IEEE 802.11g and/or IEEE 802.11n), voice over Internet Protocol (VoIP), Wi-MAX, a protocol for e-mail (e.g., Internet message access protocol (IMAP) and/or post office protocol (POP)), instant messaging (e.g., extensible messaging and presence protocol (XMPP), Session Initiation Protocol for Instant Messaging and Presence Leveraging Extensions (SIMPLE), Instant Messaging and Presence Service (IMPS)), and/or Short Message Service (SMS), or any other suitable communication protocol, including communication protocols not yet developed as of the filing date of the present disclosure.

In some embodiments, there is a single glucose sensor attached to the subject and the adherence device 200 and/or the monitor device 250 is part of the glucose sensor 102. That is, in some embodiments, the adherence device 200 and/or the monitor device 250 and the glucose sensor 102 are a single device.

In some embodiments the adherence device 200 and/or the monitor device 250 is part of an insulin pen or pump 104. That is, in some embodiments, the adherence device 200 and/or the monitor device 250 and an insulin pen or pump 104 are a single device.

Of course, other topologies of system 48 are possible. For instance, rather than relying on a communications network 106, the one or more glucose sensors 102 and the one or more insulin pens and/or pumps 104 may wirelessly transmit information directly to the adherence device 200 and/or monitor device 250. Further, the adherence device 200 and/or the monitor device 250 may constitute a portable electronic device, a server computer, or in fact constitute several computers that are linked together in a network or be a virtual machine in a cloud computing context. As such, the exemplary topology shown in Figure 1 merely serves to describe the features of an embodiment of the present disclosure in a manner that will be readily understood to one of skill in the art.

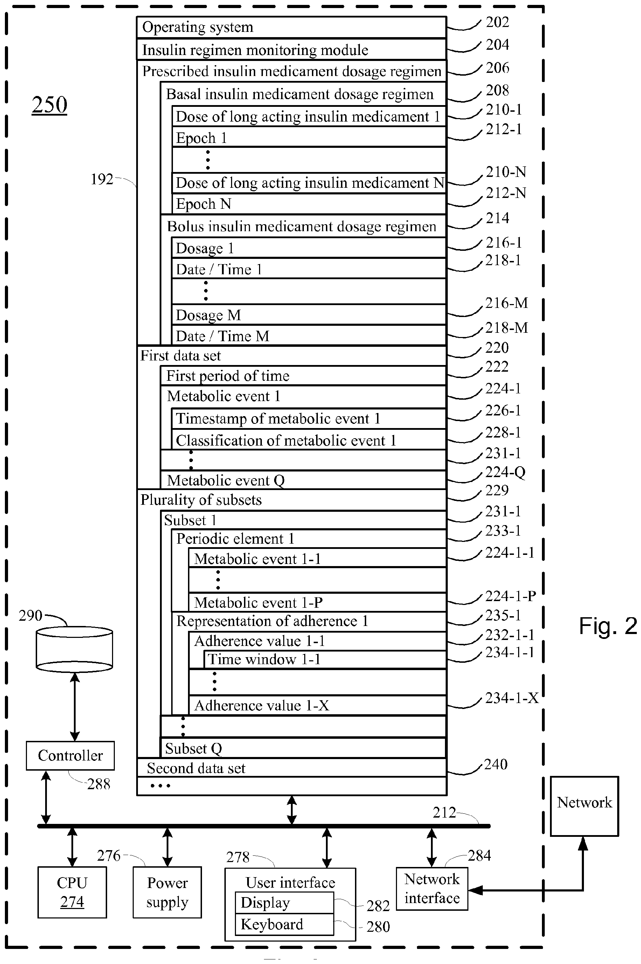

Referring to Figure 2, in typical embodiments, the monitor device 250 comprises one or more computers. For purposes of illustration in Figure 2, the monitor device 250 is represented as a single computer that includes all of the functionality for monitoring adherence to a prescribed insulin medicament dosage regimen. However, the disclosure is not so limited. The functionality for monitoring adherence to a prescribed insulin medicament dosage regimen may be spread across any number of networked computers and/or reside on each of several networked computers and/or by hosted on one or more virtual machines at a remote location accessible across the communications network 106. One of skill in the art will appreciate that a wide array of different computer topologies are possible for the application and all such topologies are within the scope of the present disclosure.

Turning to Figure 2 with the foregoing in mind, an exemplary monitor device 250 for monitoring adherence to a prescribed insulin medicament dosage regimen comprises one or more processing units (CPU's) 274, a network or other communications interface 284, a memory 192 (e.g., random access memory), one or more magnetic disk storage and/or persistent devices 290 optionally accessed by one or more controllers 288, one or more communication busses 212 for interconnecting the aforementioned components, and a power supply 276 for powering the aforementioned components. Data in memory 192 can be seamlessly shared with non-volatile memory 290 using known computing techniques such as caching. Memory 192 and/or memory 290 can include mass storage that is remotely located with respect to the central processing unit(s) 274. In other words, some data stored in memory 192 and/or memory 290 may in fact be hosted on computers that are external to the monitor device 250 but that can be electronically accessed by the monitor device 250 over an Internet, intranet, or other form of network or electronic cable (illustrated as element 106 in Figure 2) using network interface 284.

The memory 192 of the monitor device 250 for monitoring adherence to a prescribed insulin medicament dosage for a subject stores:

- an operating system 202 that includes procedures for handling various basic system services;
- an insulin regimen monitoring module 204;
- a prescribed insulin medicament dosage regimen 206 for a subject, the prescribed insulin medicament dosage regimen comprising a basal insulin medicament dosage regimen 208 and/or, optionally in some embodiments, a bolus insulin medicament dosage regimen 214;

- a first data set 220, the first data set representing a period of time 222 and comprising a plurality of metabolic events the subject engaged in during this first period of time and, for each respective metabolic event 224 in the plurality of metabolic events, a timestamp 226 representing when the respective metabolic event occurred as well as a first classification 228 of the respective metabolic event;
- a plurality of subsets 229, each respective subset 231 of the plurality of subsets 229 being the subset of metabolic events 224 for a different periodic element 233 in a plurality of periodic elements, and each respective subset 231 including a representation of adherence 235; and
- an optional second data set 240 for the subject.

In some embodiments, the insulin regimen monitoring module 204 is accessible within any browser (phone, tablet, laptop/desktop). In some embodiments the insulin regimen monitoring module 204 runs on native device frameworks, and is available for download onto the monitor device 250 running an operating system 202 such as Android or iOS.

In some implementations, one or more of the above identified data elements or modules of the monitor device 250 for monitoring adherence to a prescribed insulin medicament dosage regimen for a subject over time are stored in one or more of the previously described memory devices, and correspond to a set of instructions for performing a function described above. The above-identified data, modules or programs (e.g., sets of instructions) need not be implemented as separate software programs, procedures or modules, and thus various subsets of these modules may be combined or otherwise re-arranged in various implementations. In some implementations, the memory 192 and/or 290 optionally stores a subset of the modules and data structures identified above. Furthermore, in some embodiments the memory 192 and/or 290 stores additional modules and data structures not described above.

In some embodiments, a monitor device 250 for monitoring adherence to a prescribed insulin medicament dosage regimen 206 for a subject over time is a smart phone (e.g., an iPhone), laptop, tablet computer, desktop computer, or other form of electronic device (e.g., a gaming console). In some embodiments, the monitor device 250 is not mobile. In some embodiments, the monitor device 250 is mobile.

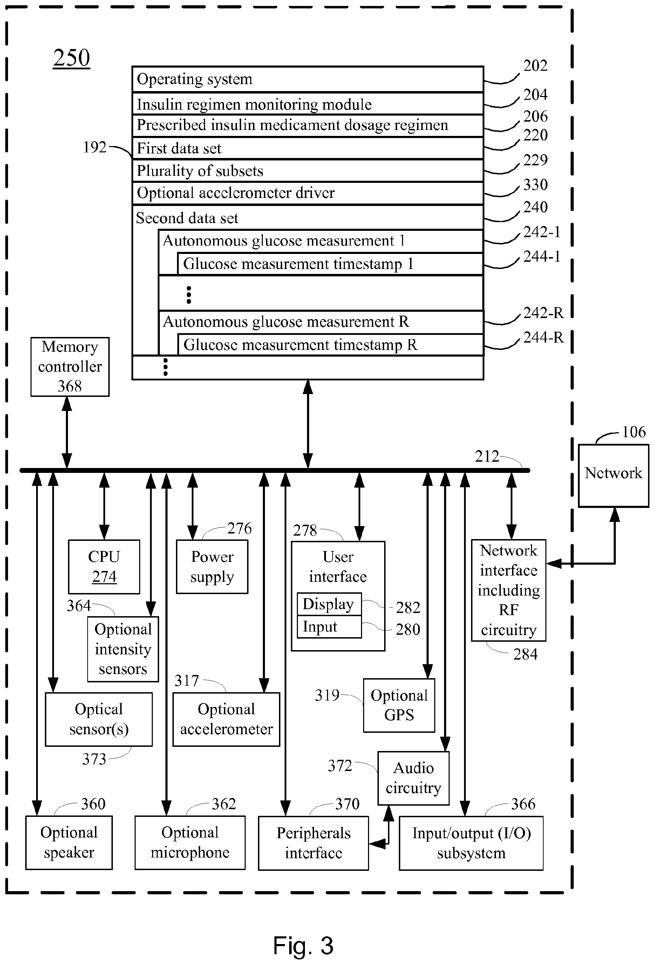

Figure 3 provides a further description of a specific embodiment of a monitor device 250 that can be used with the instant disclosure. The monitor device 250 illustrated in Figure 3 has one or more processing units (CPU's) 274, peripherals interface 370, memory controller 368, a network or other communications interface 284, a memory 192 (e.g., random access memory), a user interface 278, the user interface 278 including a display 282 and input 280 (e.g., keyboard, keypad, touch screen), an optional accelerometer 317, an optional GPS 319, optional audio circuitry 372, an optional speaker 360, an optional microphone 362, one or more optional intensity sensors 364 for detecting intensity of contacts on the monitor device 250 (e.g., a touch-sensitive surface such as a touch-sensitive display system 282 of the monitor device 250), an optional input/output (I/O) subsystem 366, one or more optional optical sensors 373, one or more communication busses 212 for interconnecting the aforementioned components, and a power system 276 for powering the aforementioned components.

In some embodiments, the input 280 is a touch-sensitive display, such as a touch-sensitive surface. In some embodiments, the user interface 278 includes one or more soft keyboard embodiments. The soft keyboard embodiments may include standard (QWERTY) and/or non-standard configurations of symbols on the displayed icons.

The monitor device 250 illustrated in Figure 3 optionally includes, in addition to accelerometer(s) 317, a magnetometer (not shown) and a GPS 319 (or GLONASS or other global navigation system) receiver for obtaining information concerning the location and orientation (e.g., portrait or landscape) of the monitor device 250 and/or for determining an amount of physical exertion by the subject.

It should be appreciated that the monitor device 250 illustrated in Figure 3 is only one example of a multifunction device that may be used for monitoring adherence to a prescribed insulin medicament dosage regimen 206 for a subject over time, and that the monitor device 250 optionally has more or fewer components than shown, optionally combines two or more components, or optionally has a different configuration or arrangement of the components. The various components shown in Figure 3 are implemented in hardware, software, firmware, or a combination thereof, including one or more signal processing and/or application specific integrated circuits.

Memory 192 of the monitor device 250 illustrated in Figure 3 optionally includes high-speed random access memory and optionally also includes non-volatile memory, such as one or more magnetic disk storage devices, flash memory devices, or other non-volatile solid-state memory devices. Access to memory 192 by other components of the monitor device 250, such as CPU(s) 274 is, optionally, controlled by the memory controller 368.

The peripherals interface 370 can be used to couple input and output peripherals of the device to CPU(s) 274 and memory 192. The one or more processors 274 run or execute various software programs and/or sets of instructions stored in memory 192, such as the insulin regimen monitoring module 204, to perform various functions for the monitoring device 250 and to process data.

In some embodiments, the peripherals interface 370, CPU(s) 274, and memory controller 368 are, optionally, implemented on a single chip. In some other embodiments, they are, optionally, implemented on separate chips.

RF (radio frequency) circuitry of network interface 284 receives and sends RF signals, also called electromagnetic signals. In some embodiments, the prescribed insulin medicament dosage regimen 206, the first data set 220, and/or the second data set 240 is received using this RF circuitry from one or more devices such as a glucose sensor 102 associated with a subject, an insulin pen or pump 104 associated with the subject and/or the adherence device 200. In some embodiments, the RF circuitry 108 converts electrical signals to/from electromagnetic signals and communicates with communications networks and other communications devices, glucose sensors 102, and insulin pens or pumps 104 and/or the adherence device 200 via the electromagnetic signals. The RF circuitry 284 optionally includes well-known circuitry for performing these functions, including but not limited to an antenna system, an RF transceiver, one or more amplifiers, a tuner, one or more oscillators, a digital signal processor, a CODEC chipset, a subscriber identity module (SIM) card, memory, and so forth. The RF circuitry 284 optionally communicates with the communication network 106. In some embodiments, the circuitry 284 does not include the RF circuitry and, in fact, is connected to the network 106 through one or more hard wires (e.g., an optical cable, a coaxial cable, or the like).

In some embodiments, audio circuitry 372, optional speaker 360, and optional microphone 362 provide an audio interface between the subject and the monitor device 250. The audio circuitry 372 receives audio data from peripherals interface 370, converts the audio data to electrical signals, and transmits the electrical signals to speaker 360. Speaker 360 converts the electrical signals to human-audible sound waves. Audio circuitry 372 also receives electrical signals converted by the microphone 362 from sound waves. Audio circuitry 372 converts the electrical signal to audio data and transmits the audio data to peripherals interface 370 for processing. Audio data is, optionally, retrieved from and/or transmitted to memory 192 and/or RF circuitry 284 by peripherals interface 370.

In some embodiments, the power supply 276 optionally includes a power management system, one or more power sources (e.g., battery, alternating current (AC)), a recharging system, a power failure detection circuit, a power converter or inverter, a power status indicator (e.g., a light-emitting diode (LED)) and any other components associated with the generation, management and distribution of power in portable devices.

In some embodiments, the monitor device 250 optionally also includes one or more optical sensors 373. The optical sensor(s) 373 optionally include charge-coupled device (CCD) or complementary metal-oxide semiconductor (CMOS) phototransistors. The optical sensor(s) 373 receive light from the environment, projected through one or more lens, and converts the light to data representing an image. The optical sensor(s) 373 optionally capture still images and/or video. In some embodiments, an optical sensor is located on the back of the monitor device 250, opposite the display 282 on the front of the device 250, so that the input 280 is enabled for use as a viewfinder for still and/or video image acquisition. In some embodiments, another optical sensor 373 is located on the front of the monitor device 250 so that the subject's image is obtained (e.g., to verify the health or condition of the subject, to determine the physical activity level of the subject, or to help diagnose a subject's condition remotely, etc.).

As illustrated in Figure 3, a monitor device 250 preferably comprises an operating system 202 that includes procedures for handling various basic system services. The operating system 202 (e.g., iOS, DARWIN, RTXC, LINUX, UNIX, OS X, WINDOWS, or an embedded operating system such as VxWorks) includes various software components and/or drivers for controlling and managing general system tasks (e.g., memory management, storage device control, power management, etc.) and facilitates communication between various hardware and software components.

In some embodiments the monitor device 250 is a smart phone. In other embodiments, the monitor device 250 is not a smart phone but rather is a tablet computer, desktop computer, emergency vehicle computer, or other form or wired or wireless networked device. In some embodiments, the monitor device 250 has any or all of the circuitry, hardware components, and software components found in the monitor device 250 depicted in Figures 2 or 3. In the interest of brevity and clarity, only a few of the possible components of the monitor device 250 are shown in order to better emphasize the additional software modules that are installed on the monitor device 250.

While the system 48 disclosed in Figure 1 can work standalone, in some embodiments it can also be linked with electronic medical records to exchange information in any way.

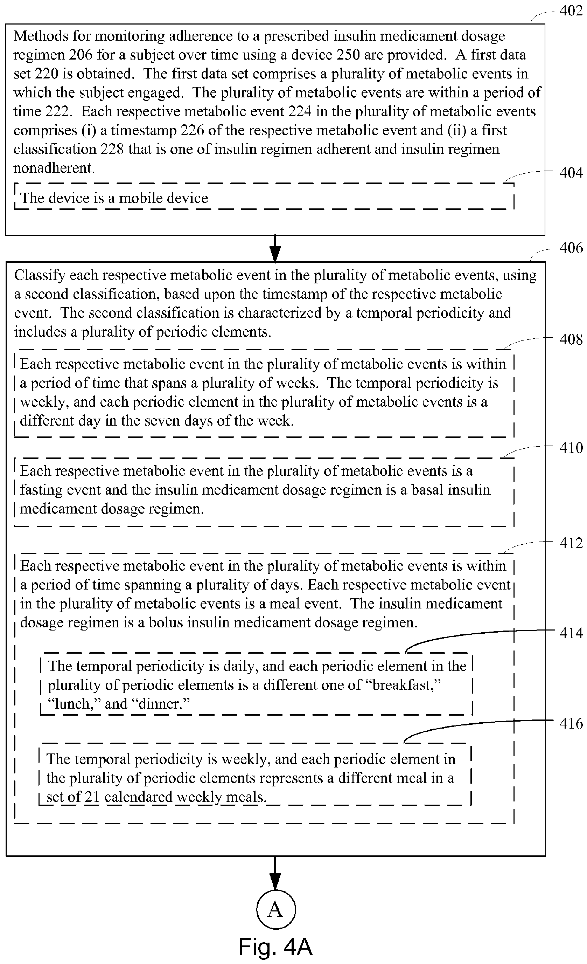

Now that details of a system 48 for monitoring adherence to a prescribed insulin medicament dosage regimen 206 for a subject over time have been disclosed, details regarding a flow chart of processes and features of the system, in accordance with an embodiment of the present disclosure, are disclosed with reference to Figures 4A through 4C. In some embodiments, such processes and features of the system are carried out by the insulin regimen monitoring module 204 illustrated in Figures 2 and 3.

*Block 402.* With reference to block 402 of Figure 4A, the goal of insulin therapy in subjects with either type 1 diabetes mellitus or type 2 diabetes mellitus is to match as closely as possible normal physiologic insulin secretion to control fasting and postprandial plasma glucose. This is done with a prescribed insulin medicament dosage regimen 206 for the subject. One aspect of the present disclosure provides a monitoring device 250 for monitoring adherence to a prescribed insulin medicament dosage regimen 206 for a subject over time. In one aspect of present disclosure, the prescribed insulin medicament dosage regimen comprises a basal insulin medicament dosage regimen 208. In another aspect of present disclosure, the prescribed insulin medicament dosage regimen comprises a bolus insulin medicament dosage regimen 214. The monitoring device comprises one or more processors 274 and a memory 192/290. The memory stores instructions that, when executed by the one or more processors, perform a method. In the method, a first data set 220 is obtained.

The first data set comprises a plurality of metabolic events in which the subject engaged. The plurality of metabolic events is within a period of time 222. In varying embodiments, this period of time 222 is one day or more, three days or more, five days or more, ten days or more, one month or more, two months or more, three months or more or five months or more. Each respective metabolic event 224 in the plurality of metabolic events comprises (i) a timestamp 226 of the respective metabolic event and (ii) a first classification 228 that is one of insulin regimen adherent and insulin regimen nonadherent.

In some embodiments each metabolic event 224 in the first data set 220 has one or more first classifications 228 set forth in Table 1.

*Table 1. Exemplary first classification 228 of metabolic events 224.*

| Category A | | | |
|---|---|---|---|
| | | A1 | In Bolus Adherence |
| | | A2 | Out of Bolus Adherence |
| Category B | | | |
| | | B1 | In Basal Adherence |

(continued)

| Category B | | | |
|---|---|---|---|
| | | B2 | Out of Basal Adherence |
| Category C | | | |
| | | C1 | In timing adherence |
| | | C2 | Out of timing adherence |
| Category D | | | |
| | | D1 | In size adherence |
| | | D2 | Out of size adherence |

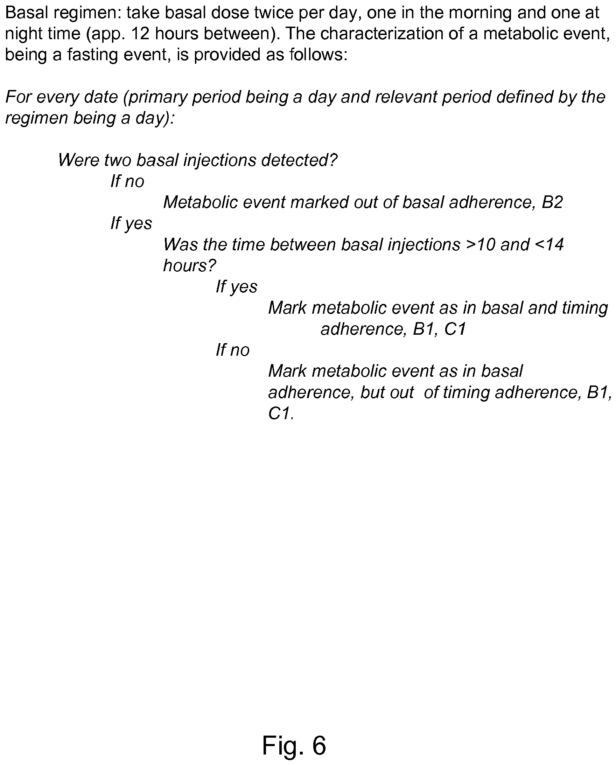

Using the first classifications set forth in Table 1, the same period of time can contain metabolic events with different labels. For instance, a whole day can contain a metabolic event (fasting event) marked as out of basal adherence, B2, but three metabolic events (meal events) within that day can be labelled in bolus adherence, A1. Figure 6 illustrates an algorithm for classifying a metabolic event, wherein the example is a fasting event, and wherein the relevant period of time defined by the regimes is one day. The classification is provided in accordance with the categories of Table 1. In such embodiments, continuously marked periods, e.g. a day, contains a fasting event marked with B2 or a meal event marked with A1, are referred to as metabolic events that have been classified according to the first classification. As another example, consider the case where three fasting events within each of the first three days of a period of one week are marked as in 100 % basal adherence (e.g. basal and timing adherence B1, C1), two fasting events within each of the two following days as in 50% basal adherence (e.g. in basal adherence but out of timing adherence B1, C2), and two fasting event within the last two days as in 0% basal adherence (out of basal adherence and out of timing adherence B2, C2). In the example where a fasting event is classified and marked as in basal and timing adherence the event can as an example be defined as 100% insulin regimen adherent, in the case where the metabolic event is marked in basal adherence, but out of timing adherence the event can as an example be defined as 50% insulin regimen adherent, this could be a different percentage, based on estimated effect of taking a dose later than recommended. In the case where the fasting event is out of basal adherence the event is 0% insulin regimen adherent corresponding to insulin regimen nonadherent. The number of insulin regimen adherent metabolic events in the example is thus 3+2*50%+2*0%. In this example the past week's adherence is thus:

$$\text{Past 7 days}'\text{ adherence} = \frac{3 + 0.5 * 2}{7} = \frac{4}{7} = 57\%$$

In another example such an adherence, or primary adherence value, can be calculated for each of the subsets 231 in the plurality of subsets 229 of the plurality of metabolic events, wherein each respective subset 231 of the plurality of metabolic events in the plurality of subsets is for different periodic elements in the plurality of periodic elements. Therefore in some embodiments of the disclosure, the method further comprises computing a plurality of primary adherence values, wherein each respective primary adherence value in the plurality of primary adherence values represents a corresponding periodic element in the plurality of periodic elements, and each respective primary adherence value in the plurality of primary adherence values is computed by dividing a number of insulin regimen adherent metabolic events in each respective subset by a total number of metabolic events in the respective subset corresponding to the respective periodic element, and wherein the respective representation of adherence for each respective subset in the plurality of subsets is collectively represented as the corresponding primary adherence value. For example consider a periodic element being a week, and a period of time being 7 weeks. In this example the periodic element (e.g. periodic element for Mondays) contains 7 metabolic events, 3 metabolic events being 100% regimen adherent, and 2 events being 50% regimen adherent, and 2 events being 0% regimen adherent, can be collectively represented as 57%.

In other embodiments, such classifications are imposed by considering metabolic events to be fasting events or meal events and classifying each fasting event or meal event for insulin medicament regimen adherence.

In some embodiments, metabolic events can be a metabolic events defined in the medicament regimen, which can be automatically identified from a device continuously measuring an indicator of an event, wherein the event is relating to a metabolic state of the subject, whereby the device allows the metabolic event to be timestamped and to be classified with respect to the medicament regimen as regimen adherent or regimen nonadherent. For example, a metabolic event defined according to the medicament regimen could be a meal event, wherein the medicament regimen determines that bolus insulin should be administered based on glucose measurements relating to this event, or it could be a fasting event, wherein the medicament regimen determines that basal insulin should be administered based on glucose measurements relating to this event.

In some embodiments, metabolic events (e.g., meal events, fasting events, etc.) incurred by the subject are identified without reliance on records kept by the subject. For instance, in some embodiments a second data set 240 comprising autonomous glucose measurements 242 of the subject from one or more glucose sensors 102 is obtained. Figure 3 illustrates. Each such autonomous glucose measurement 242 is timestamped with a glucose measurement timestamp 244 to represent when the respective measurement was made.

The FREESTYLE LIBRE CGM by ABBOTT ("LIBRE") is an example of a glucose sensor that may be used as a glucose sensor 102. The LIBRE allows calibration-free glucose measurements with an on-skin coin-sized sensor, which can send up to eight hours of data to a reader device (e.g., the adherence device 200 and/or the monitor device 250) via near field communications, when brought close together. The LIBRE can be worn for fourteen days in all daily life activities. In some embodiments, autonomous glucose measurements are taken from the subject at an interval rate of 5 minutes or less, 3 minutes or less, or 1 minute or less. Example 1 below illustrates how such autonomous glucose measurements are used to both identify metabolic events and to classify each of them as insulin regimen adherent or insulin regimen nonadherent.

Referring to block 404 of Figure 4A, advantageously, the first data set 220 can be communicated to a monitor device 250 that is mobile to thereby monitor adherence to a prescribed insulin medicament dosage regimen for the subject over time. Thus, in some embodiments, the monitor device 250 is a mobile device.

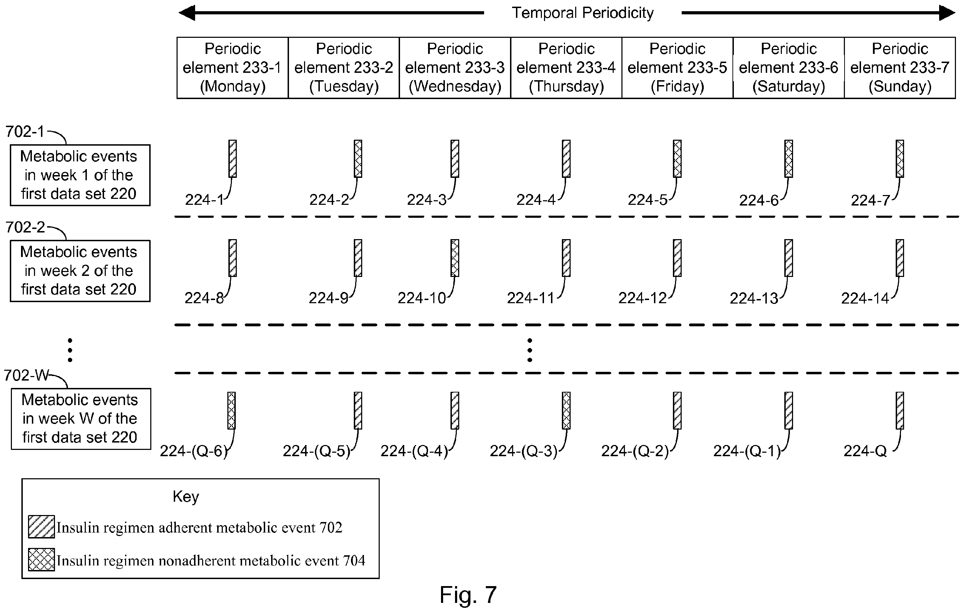

*Block 406.* Referring to block 406 of Figure 4A, the method continues by classifying each respective metabolic event 224 in the plurality of metabolic events, using a second classification, based upon the timestamp 226 of the respective metabolic event. The second classification has a temporal periodicity and includes a plurality of periodic elements. The embodiment illustrated in block 408 of Figure 4A provides an example of this second form of classification. Each respective metabolic event 224 in the plurality of metabolic events is within a period of time that spans a plurality of weeks. In other words, the period of time 222 encompassed by the first data set 220 is a number of weeks (e.g., three or more weeks, five or more weeks, or ten or more weeks). The temporal periodicity is weekly, and each periodic element 233 in the plurality of metabolic events is a different day in the seven days of the week. This is further illustrated in Figure 7. In Figure 7, the temporal periodicity specified by the second classification (e.g., weekly) is used to divide the metabolic events 224 into weeks 702, and then each respective metabolic event is arranged according to its respective timestamp 226 into a periodic element 233. For instance, in Figure 7, the first week 702-1 of metabolic events 224 in the first data set consists of metabolic events 224-1 through 224-7, and each of these metabolic events fall on a different day of the week according to their respective timestamps 226. Accordingly, metabolic events 224-1 through 224-7 are respectively classified into periodic elements 233-1 through 233-7 as illustrated in Figure 7. Further, the second week 702-2 of metabolic events 224 in the first data set consists of metabolic events 224-8 through 224-14, and each of these metabolic events fall on a different day of the week according to their respective timestamps 226. Accordingly, metabolic events 224-8 through 224-14 are respectively categorized into periodic elements 233-1 through 233-7 as illustrated in Figure 7. This second classification proceeds through the first data set so that the $w^{th}$ week 702-W of metabolic events 224 in the first data set, consisting of metabolic events 224-(Q-6) through 224-Q, are respectively classified into periodic elements 233-1 through 233-7 as illustrated in Figure 7.

While Figure 7 illustrates an example in which each respective period of data in the first data set (e.g., the metabolic events of period 702-1, the metabolic events of period 702-2, and so forth) includes a metabolic event 224 for each periodic element 233, the present disclosure is not so limited. In some embodiments, each respective period of data in the first data set (e.g., the metabolic events of period 702-1, the metabolic events of period 702-2, and so forth) includes two or more metabolic events 224 for a periodic element 233, includes three or more metabolic events 224 for a periodic element 233, or includes four or more metabolic events 224.

While Figure 7 illustrates an example in which each respective period of data in the first data set (e.g., the metabolic events of period 702-1, the metabolic events of period 702-2, and so forth) includes the same number of metabolic events 224 for each respective periodic element 233 (e.g., exactly one metabolic event 224 per periodic element 233 per period), the present disclosure is not so limited. In some embodiments, each respective period of data in the first data set 220 (e.g., the metabolic events of period 702-1, the metabolic events of period 702-2, and so forth) includes an independent number (the same or a different number) of metabolic events 224 for each respective periodic element 233. For instance, in some embodiments, the metabolic events 224 for the first period 702-1 may include one metabolic event 224 for the first periodic element 233-1 (Monday) and two metabolic events 224 for the second periodic element 233-2 (Tuesday).

While Figure 7 illustrates an example in which each respective period of data in the first data set 220 (e.g., the metabolic events of period 702-1, the metabolic events of period 702-2, and so forth) includes at least one metabolic event 224 for each periodic element 233 (e.g., exactly one metabolic event 224 per periodic element 233 per period), the present disclosure is not so limited. In some embodiments, a respective period of data in the first data set 220 (e.g., the metabolic events of period 702-1) includes no metabolic events 224 for a particular periodic element 233. For instance, in some embodiments, the metabolic events 224 for the first period 702-1 may include zero metabolic events 224 for the first periodic element 233-1 (Monday) and one metabolic event 224 for the second periodic element 233-2 (Tuesday).

As illustrated in Figure 7, each of the metabolic events 224 in the first data set is already classified in accordance with a first classification which is one of "insulin regimen adherent" 702 and "insulin regimen nonadherent" 704. This is an example of a first classification 228 applied to each of the metabolic events 224. Referring to block 410 of Figure 4A, in some such embodiments, each respective metabolic event 224 in the plurality of metabolic events is a fasting event and the prescribed insulin medicament dosage regimen 206 is a basal insulin medicament dosage regimen 208.

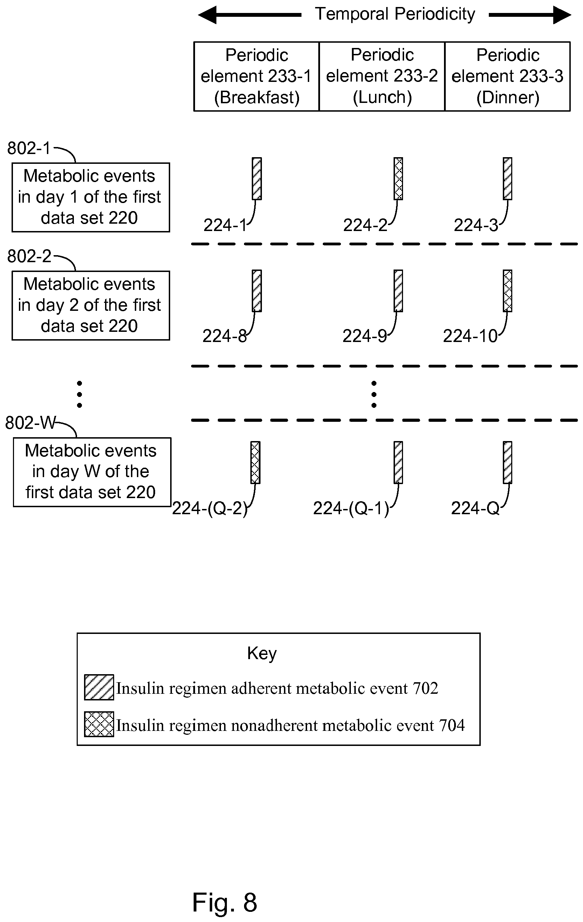

Referring to block 412 of Figure 4A, in some embodiments, each respective metabolic event 224 in the plurality of metabolic events is within a period of time spanning a plurality of days. Further, each respective metabolic event 224 in the plurality of metabolic events is a meal event and the prescribed insulin medicament dosage regimen 206 is a bolus insulin medicament dosage regimen 214. Referring to block 414 of Figure 4A, in some such embodiments, the temporal periodicity is daily, and each periodic element in the plurality of periodic elements is a different one of "breakfast," "lunch," and "dinner." Figure 8 illustrates, in Figure 8, the temporal periodicity specified by the second classification (e.g., daily) is used to divide the metabolic events 224 into days 702, and then each respective metabolic event is arranged according to its respective timestamp 226 into a periodic element 233. For instance, in Figure 8, the first day 802-1 of metabolic events 224 in the first data set 220 consists of metabolic events 224-1 through 224-3, and each of these metabolic events are classified into a different meal of the day according to their respective timestamps 226. Accordingly, metabolic events 224-1 through 224-3 are respectively classified into periodic elements 233- 1 through 233-3 ("Breakfast," "Lunch," and "Dinner") as illustrated in Figure 8. Further, the second day 802-2 of metabolic events 224 in the first data set 220 consists of metabolic events 224-4 through 224-6, and each of these metabolic events are classified into a different meal of the day according to their respective timestamps 226. Accordingly, metabolic events 224-4 through 224-6 are respectively classification into periodic elements 233-1 through 233-3 as illustrated in Figure 8. This second classification proceeds through the first data set 220 so that the $w^{th}$ day 802-W of metabolic events 224 in the first data set, consisting of metabolic events 224-(Q-2) through 224-Q, are respectively categorized into periodic elements 233-1 through 233-3 as illustrated in Figure 8.

Block 416 of Figure 4A illustrates yet another embodiment of how the respective metabolic events 224 of the first data set 220 are classified using a second classification, based upon the timestamps 226 of the respective metabolic events. Here, the temporal periodicity is weekly, and each periodic element in the plurality of periodic elements represents a different meal in a set of 21 calendared weekly meals. Thus, the set of periodic elements in this second classification consists of 21 periodic elements, whereas the set of periodic elements in the embodiment illustrated in Figure 7 consists of 7 periodic elements, and the set of periodic elements in the embodiment illustrated in Figure 8 consists of 3 periodic elements.

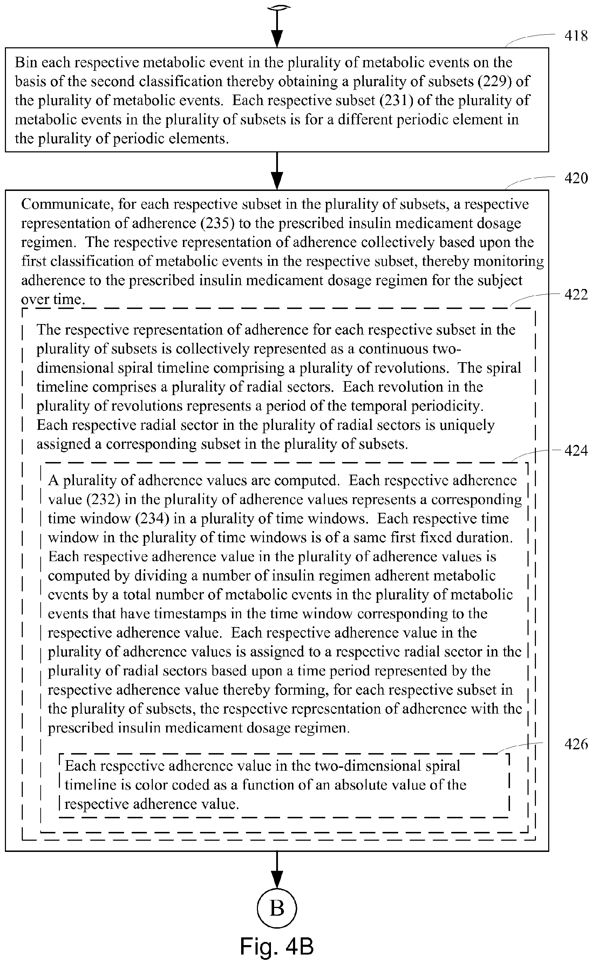

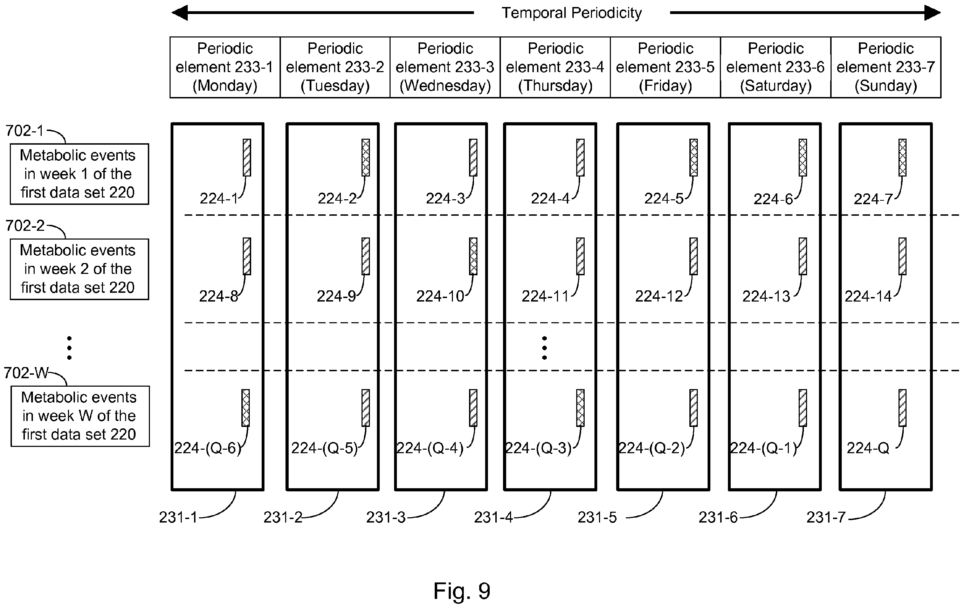

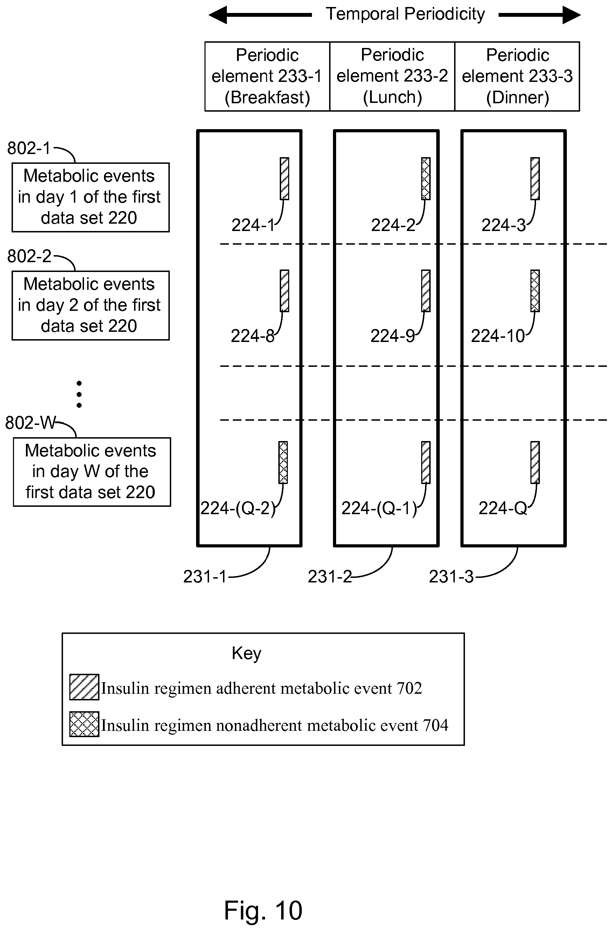

Referring to block 418 of Figure 4B, the process continues by binning each respective metabolic event 224 in the plurality of metabolic events on the basis of the second classification thereby obtaining a plurality of subsets 229 of the plurality of metabolic events. Each respective subset 231 of the plurality of metabolic events in the plurality of subsets is for a different periodic element 233 in the plurality of periodic elements. As one example of this binning, in the embodiment of the first data set 220 classified according to the scheme illustrated in Figure 7, subsets 231 illustrated in Figure 9 are formed upon binning each respective metabolic event 224 in the plurality of metabolic events of Figure 7 on the basis of the second classification. As another example, in the embodiment of the first data set 220 classified according to the schemed illustrated in Figure 8, subsets 231 illustrated in Figure 10 are formed upon binning each respective metabolic event 224 in the plurality of metabolic events of Figure 8 on the basis of the second classification. Further, Figure 2 illustrates a data structure 229 that is formed, upon such binning, according to one embodiment of the present disclosure. The plurality of subsets 229 includes, for each respective subset 231, a representation of each respective periodic element 233 in the plurality of periodic elements of the second classification, and for each respective periodic element 233, a representation of the metabolic events 224 categorized into the respective periodic element 233 for the respective subset 231.

*Block 420.* Referring to block 420 on Figure 4B, the process continues with the communication, for each respective subset 231 in the plurality of subsets 229, a respective representation of adherence 235 to the prescribed insulin medicament dosage regimen. In some embodiments, this respective representation of adherence is collectively based upon the first classification of metabolic events in the respective subset. In this way, adherence to the prescribed insulin medicament dosage regimen 206 for the subject over time is accomplished.

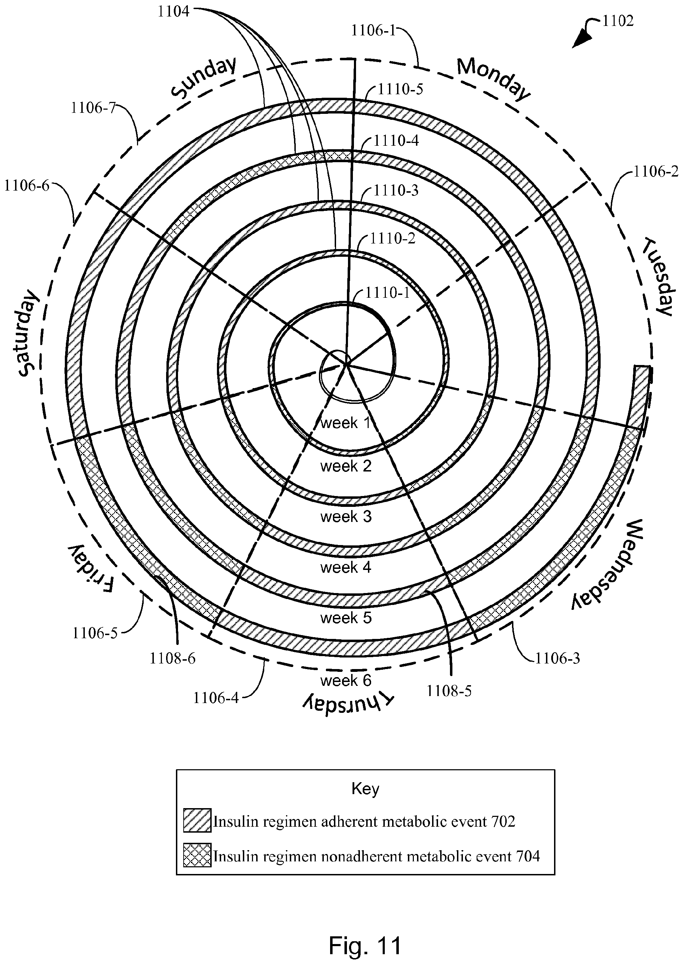

Referring to block 422, and as further illustrated in Figure 11, in some embodiments, the respective representation of adherence 235 for each respective subset 231 in the plurality of subsets 229 is collectively represented as a continuous two-dimensional spiral timeline 1102 comprising a plurality of revolutions. The spiral timeline 1102 comprises a plurality of radial sectors 1106. Each revolution 1104 in the plurality of revolutions represents a period of the temporal periodicity. Each respective radial sector 1106 in the plurality of radial sectors is uniquely assigned a corresponding subset 231 in the plurality of subsets 229.

In the case of Figure 11, the subsets illustrated in Figure 9 are mapped onto the continuous two-dimensional spiral timeline 1102. In this example, each revolution 1104 in the plurality of revolutions represents a week 702. Each respective radial sector 1106 in the plurality of radial sectors corresponding to a subset 229 in the plurality of subsets 231, and thus represents a day of the week in this example. In some embodiments, each respective portion of the revolution 1104 in each radial sector 1106 is marked in accordance with the first classification 228 of the metabolic events 224 that fall onto the respective portion of the revolution. For instance, referring to Figure 11, if the metabolic events 224 that fall onto a respective portion 1108-5 of a revolution 1104 within a sector 1106-4 of the continuous two-dimensional spiral timeline 1102 (e.g., Thursday, week 5) have a first classification 228 of "insulin regimen adherent," then the respective portion 1108-5 of the revolution 1104 is marked "insulin regimen adherent." On the other hand, if the metabolic events 224 that fall onto a respective portion 1108-6 of a revolution 1104 of the continuous two-dimensional spiral timeline 1102 (e.g., Friday, week 6) have a first classification 228 of "insulin regimen nonadherent," then the respective portion 1108-6 of the revolution 1104 is marked "insulin regimen nonadherent."

In some embodiments, if there is more than one metabolic event 224 that falls into a respective portion of a revolution 1104 within a sector 1106 of the continuous two-dimensional spiral timeline 1102, then different schemes may be used to represent such classifications. In some embodiments, each respective metabolic event is represented on a portion of the revolution that temporally represents the respective metabolic event. For instance, the shading of the portion of the revolution may correspond to the first classification 228 of the metabolic event 224 similar to that shown in Figure 11.

Alternatively, the first classification of each of the metabolic events 224 that fall into a respective portion of a revolution 1104 within a sector 1106 of the continuous two-dimensional spiral timeline 1102 may be combined into a single adherence value 234 which is then represented on the respective portion of the revolution 1104 within a sector 1106. Block 424 of Figure 4B describes such an embodiment.

Referring to block 424 of Figure 4B, in some embodiments, a plurality of adherence values 232 is computed. Each respective adherence value 232 in the plurality of adherence values represents a corresponding time window 234 in a plurality of time windows. Thus, referring to Figure 11, each respective portion of a revolution 1104 within a sector 1106 of the continuous two-dimensional spiral timeline 1102 is a time window 234. Thus, the five time windows for Monday are portions 1110-1 through 1110-5 respectively.

In some embodiments, each respective time window in the plurality of time windows is of a same first fixed duration (e.g., 1 week as illustrated in Figure 11, 1 day, one month, or a number of hours). Each respective adherence value 232 in the plurality of adherence values is computed by dividing a number of insulin regimen adherent metabolic events by a total number of metabolic events in the plurality of metabolic events that have timestamps in the time window corresponding to the respective adherence value. In some embodiments, each respective adherence value in the plurality of adherence values is assigned to a portion of a revolution 1104 within a radial sector 1106 in the plurality of radial sectors based upon a time period represented by the respective adherence value thereby forming, for each respective subset in the plurality of subsets, the respective representation of adherence with the prescribed insulin medicament dosage regimen. Thus, using Figure 11 to illustrate, the first classifications 228 of the metabolic events 224 falling on Monday of week 1 are used to calculate an adherence value 232-1 and this adherence value is assigned to radial sector 1110-1, the first classifications 228 of the metabolic events 224 falling on Monday of week 2 are used to calculate an adherence value 232-2 and this adherence value is assigned to radial sector 1110-2, and so forth.

In some embodiments the first classification 228 of all the metabolic events within a radial sector 1106 are used collectively to compute a single adherence value for the entire sector and the entire sector is colored or marked based upon a value of this single adherence value 232. In such embodiments, each respective time window in the plurality of time windows is of a same first fixed duration (e.g., 1 week as illustrated in Figure 11, 1 day, one month, or a number of hours). Each respective adherence value 232 in the plurality of adherence values is computed by dividing a number of insulin regimen adherent metabolic events (e.g., the insulin regimen adherent metabolic events falling on a Monday) by a total number of metabolic events in the plurality of metabolic events that have timestamps in the time window corresponding to the respective adherence value (e.g., all the metabolic events falling on a Monday). In some embodiments, each respective adherence value in the plurality of adherence values is assigned the radial sector 1106 in the plurality of radial sectors based upon a time period represented by the respective adherence value thereby forming, for each respective subset in the plurality of subsets, the respective representation of adherence with the prescribed insulin medicament dosage regimen. Thus, using Figure 11 to illustrate, the first classifications 228 of the metabolic events 224 falling on any Monday are used to calculate an adherence value 232-1 and this adherence value is assigned to sector 1106-1.

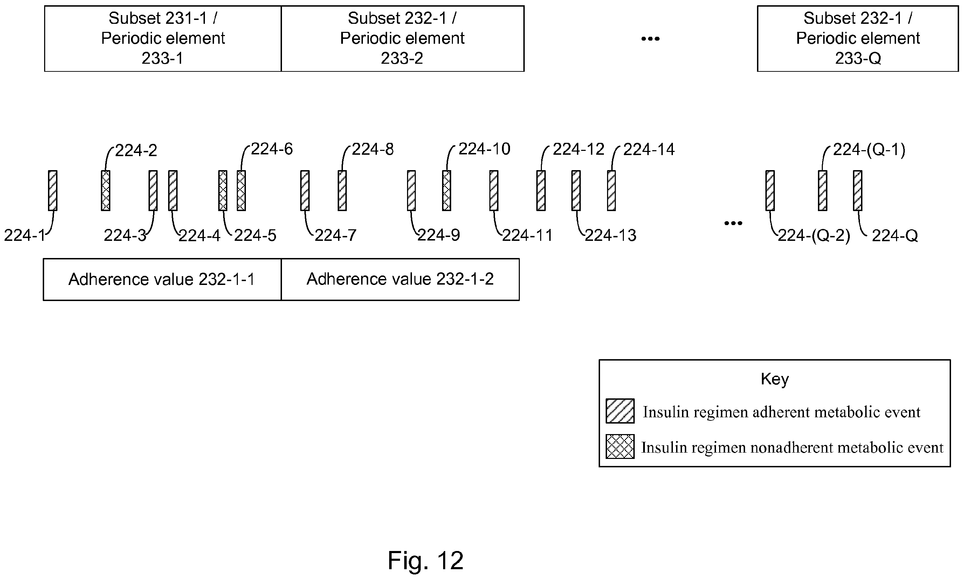

In some embodiments, each adherence value 232 is computed by dividing a number of insulin regimen adherent metabolic events for a periodic element 231-1 within a subset 231 (e.g., Mondays occurring within the subset, "Breakfast," etc.) by a total number of metabolic events for the periodic 233 element in the subset 231. For example, consider the subset 231-1 of Figure 12 in which there are two insulin regimen adherent metabolic events (224-1 and 224-3) and one insulin regimen nonadherent metabolic event for a total of three metabolic events 224 for the periodic element 233-1 in the subset 231-1. In this example, the adherence value 232-1-1 is computed by dividing the number of insulin regimen adherent metabolic events for the periodic element 233-1 in the subset 231-1 (two, 224-1 and 224-3) by the total number of metabolic events for the periodic element 233-1 in the subset 231-1 (three, 224-1, 224-2, and 224-3), that is dividing "2" by "3." It will be appreciated that the process of dividing a number of insulin regimen adherent metabolic events by a total number of metabolic events can be done any number of ways and all such ways are encompassed in the present disclosure. For instance, the division can be effectuated by, in fact, multiplying a number of insulin regimen adherent metabolic events by the inverse of the total number of metabolic events (e.g., in the example above, by computing (2 * (1/3)).

In some embodiments, each adherence value 231 is computed by dividing a number of insulin regimen adherent metabolic events for a periodic element 233-1 in a subset 231 by a total number of metabolic events for the periodic element in the subset. For example, consider the subset 231-1 of Figure 12 in which there are three insulin regimen adherent metabolic events (224-1, 224-3 and 224-4) and three insulin regimen nonadherent metabolic events for a total of six metabolic events 224 for the periodic element 233-1 for the subset 231-1. In this example, the adherence value 232-1 is computed by dividing the number of insulin regimen adherent metabolic events for the periodic element 233-1 in the subset 231-1 (three, 224-1, 224-3 and 224-4) by the total number of metabolic events for the periodic element 233 for the subset 231-1 (six, 224-1, 224-2, 224-3, 224-4, 224-5 and 224-6), that is dividing "3" by "6." It will be appreciated that the process of dividing a number of insulin regimen adherent metabolic events by a total number of metabolic events can be done any number of ways and all such ways are encompassed in the present disclosure. For instance, the division can be effectuated by, in fact, multiplying a number of insulin regimen adherent metabolic events by the inverse of the total number of metabolic events (e.g., in the example above, by computing (3 * (1/6)).

In some embodiments, calculated adherence values 232 are scaled so that they fall into a range other than their native range. Thus, in some embodiments, the native range of the calculated adherence values 232 is zero to 1, but they are then uniformly scaled to zero to 100, zero to 1000, or any other suitable scale. Such scaling acts independently of any down weighting of metabolic events 224.

In some embodiments, the first classification 228 of respective metabolic events 224 that occur earlier than a set cutoff time are down-weighted relative to respective metabolic events in the plurality of metabolic events that occur after the set cutoff time. In some embodiments, metabolic events occurring before the set cutoff time are down weighted as a function of time, so that events occurring earlier in time than later events are down weighted more.

Referring to block 426 of Figure 4B, in some embodiments, each respective adherence value in the two-dimensional spiral timeline 1102 is color coded as a function of an absolute value of the respective adherence value. As discussed in Example 2, it is often the case that adherence values will fall into a range between zero and one. Thus, in accordance with block 426, a color table can be used to convert this range into a color (e.g., low numbers are red shifted and higher number are green or blue shifted) and used to color the corresponding portion of a revolution 1104 within a radial sector 1106 in the plurality of radial sectors or the entire radial sector 1106.

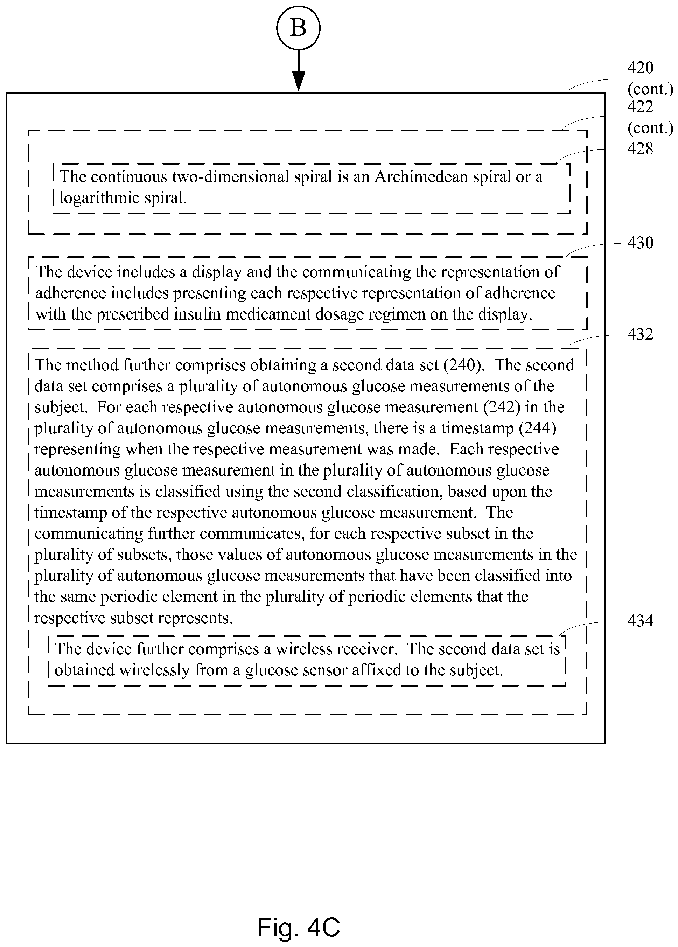

Such display allows for a user to ascertain which periodic elements have poor adherence. For instance, the disclosed systems and methods allow a user to discover trends in regimen adherence, such as a particular day of the week, time of the month, meal in the day, or meal in the week has more regimen adherence. Referring to block 428 of Figure 4C, in some embodiments, the continuous two-dimensional spiral 1102 is an Archimedean spiral or a logarithmic spiral.

Referring to block 430, in some embodiments the device 250 includes a display and the communicating the representation of adherence includes presenting each respective representation of adherence with the prescribed insulin medicament dosage regimen on the display. Moreover, in some embodiments, the user can rescale the periodicity, for instance dynamically switching between the set of periodic elements "Breakfast," "Lunch," and "Dinner," to the days of the week in order to identify periodic regimen nonadherence trends.

Referring to block 432 of Figure 4C, in some embodiments, the method further comprises obtaining a second data set 240. The second data set comprises a plurality of autonomous glucose measurements of the subject and, for each respective autonomous glucose measurement 242 in the plurality of autonomous glucose measurements, there is a timestamp 244 representing when the respective measurement was made. Each respective autonomous glucose measurement in the plurality of autonomous glucose measurements is classified using the second classification, based upon the timestamp of the respective autonomous glucose measurement. In such embodiments, the communicating further communicates, for each respective subset in the plurality of subsets, those values of autonomous glucose measurements in the plurality of autonomous glucose measurements that have been classified into the same periodic element in the plurality of periodic elements that the respective subset represents. In some embodiments, the glucose data is temporally matched to the representations of adherence and shown in a single display. In some such embodiments, the monitor device 250 comprises a wireless receiver 284 and the second data set is obtained wirelessly from a glucose sensor affixed to the subject.

In some embodiments, the adherence device 250 allows a subject to add and mark events manually which are then displayed temporally within or the representation of adherence, or beside it. In some such embodiments, the adherence device 250 suggests categories for the subject to choose from, e.g. events such as meals, insulin and glucose measurements, sleeping periods, periods of physical activity, sick days. In some embodiments, these events are marked with a specific category name, which is then used to identify causes of poor glycaemic control and provide improved treatment transparency. For instance, in some embodiments this is accomplished by temporally superimposing these additional events onto the representation of adherence and displaying the superposition on the display of the monitor device 250. In some embodiments, these additional events are detected by a wearable device.

*Example 1: Use of autonomous glucose measurements to identify metabolic events and to classify them as insulin regimen adherent or insulin regimen nonadherent.* Block 402 above described how a second data set 240 comprising a plurality of glucose measurements is obtained autonomously in some embodiments. In this example, in addition to the autonomous glucose measurements, insulin administration events are obtained in the form of insulin medicament records from one or more insulin pens and/or pumps 104 used by the subject to apply the prescribed insulin regimen. These insulin medicament records may be in any format, and in fact may be spread across multiple files or data structures. As such, in some embodiments, the instant disclosure leverages the recent advances of insulin administration pens, which have become "smart" in the sense that they can remember the timing and the amount of insulin medicament administered in the past. One example of such an insulin pen 104 is the NovoPen 5. Such pens assist patients in logging doses and prevent double dosing. It is contemplated that insulin pens will be able to send and receive insulin medicament dose volume and timing, thus allowing the integration of continuous glucose monitors 102, insulin pens 104 and the algorithms of the present disclosure. As such, insulin medicament records from one or more insulin pens 104 and/or pumps is contemplated, including the wireless acquisition of such data from the one or more insulin pens 104.

In some embodiments, each insulin medicament record comprises: (i) a respective insulin medicament injection event including an amount of insulin medicament injected (or pumped) into the subject using a respective insulin pen in the one or more insulin pens and (ii) a corresponding electronic timestamp that is automatically generated by the respective insulin pen 104 or pump upon occurrence of the respective insulin medicament injection event.

In some embodiments, a plurality of fasting events, which is one form of metabolic event 224, are identified using the autonomous glucose measurements 242 of the subject and their associated glucose measurement timestamps 244 in the second data set 240. Glucose measurements during fasting events are of importance for measuring basal glucose levels.

There are a number of methods for detecting a fasting event using autonomous glucose measurements 242 from a glucose monitor 102. For instance, in some embodiments, a first fasting event (in the plurality of fasting events) is identified in a first time period (e.g., a period of 24 hours) encompassed by the plurality of autonomous glucose measurements by first computing a moving period of variance $\sigma_k^2$ across the plurality of autonomous glucose measurements, where:

$$\sigma_k^2 = \left(\frac{1}{M}\sum_{i=k-M}^{k}(G_i - \overline{G})\right)^2$$

and where, $G_i$ is the $i^{th}$ glucose measurement in the portion k of the plurality of glucose measurements, M is a number of glucose measurements in the plurality of glucose measurements and represents a contiguous predetermined time span, $\overline{G}$ is the mean of the M glucose measurements selected from the plurality of glucose measurements, and k is within the first time period. As an example, the glucose measurements may span several days or weeks, with autonomous glucose measurements taken every five minutes. A first time period k (e.g., one day) within this overall time span is selected and thus the portion k of the plurality of measurements is examined for a period of minimum variance. The first fasting period is deemed to be the period of minimum variance $\min_k \sigma_k^2$ within the first time period. Next, the process is repeated with portion k of the plurality of glucose measurements by examining the next portion k of the plurality of glucose measurements for another period of minimum variance thereby assigning another fasting period. Repetition of this method through all portions k of the plurality of glucose measurements is used to build the plurality of fasting periods.

Once the fasting events are identified, by the method described above or any other method, a first classification 228 is applied to each respective fasting event in the plurality of identified fasting events. Thus, for each respective fasting event there is a first classification 228 for the respective fasting event. The first classification is one of insulin regimen adherent and insulin regimen nonadherent. More specifically, here, the first classification is one of basal insulin regimen adherent and basal insulin regimen nonadherent.

A respective fasting event is deemed basal insulin regimen adherent when the acquired one or more medicament records establish, on a temporal and quantitative basis, adherence with the prescribed basal insulin medicament dosage regimen during the respective fasting event. A respective fasting event is deemed basal regimen nonadherent when the acquired one or more medicament records do not include one or more medicament records that establish, on a temporal and quantitative basis, adherence with the prescribed basal insulin medicament dosage regimen during the respective fasting event. In some embodiments the basal insulin medicament dosage regimen 208 specifies that a basal dose of long acting insulin medicament 210 is to be taken during each respective epoch 212 in a plurality of epochs and that a respective fasting event is deemed basal insulin medicament regimen 208 nonadherent when there are no medicament records for the epoch 212 associated with the respective fasting event. In various embodiments, each epoch in the plurality of epochs is two days or less, one day or less, or 12 hours or less. Thus, consider the case where the second data set 240 is used to identify a fasting period and the prescribed basal insulin medicament dosage regimen 208 specifies to take dosage A of a long acting insulin medicament 210 every 24 hours. In this example, therefore, the epoch is one day (24 hours). The fasting event is inherently timestamped because it is derived from a period of minimum variance in timestamped glucose measurements, or by other forms of analysis of the timestamped autonomous glucose measurements.

Thus, in some embodiments the timestamp, or period of fasting, represented by a respective fasting event is used as a starting point for examining whether the fasting event is basal insulin medicament regimen adherent. For instance, if the period of fasting associated with the respective timestamp is 6:00 AM on Tuesday, May 17, what is sought in the medicament injection records is evidence that the subject took dosage A of the long acting insulin medicament in the 24 hour period (the epoch) leading up to 6:00 AM on Tuesday, May 17 (and not more or less of the prescribed dosage). If the subject took the prescribed dosage of the long acting insulin medicament during this epoch, the respective fasting event (and/or the basal injection event and/or the glucose measurements during this time) is deemed basal regimen adherent. If the subject did not take the prescribed dosage of the long acting insulin medicament 210 during this epoch 212 (or took more than the prescribed dosage of the long acting insulin medicament during this period), the respective fasting event (and/or the basal injection event and/or the glucose measurements during this time) is deemed basal regimen nonadherent.

While the use of the fasting event to retrospectively determine whether a basal injection event is basal insulin medicament regimen adherent, the present disclosure is not so limited. In some embodiments, the epoch is defined by the basal insulin medicament regimen and, so long as the subject took the amount of basal insulin required by the basal regimen during the epoch (and not more), even if after the fasting event, the fasting event will be deemed basal insulin medicament regimen adherent. For instance, if the epoch is one day beginning each day at just after midnight (in other words the basal regimen specifies one or more basal insulin medicament dosages to be taken each day, and further defines a day as beginning and ending at midnight), and the fasting event occurs at noon, the fasting event will be deemed compliant provided that the subject takes the basal injections prescribed for the day at some point during the day.

In some embodiments, a fasting event is not detected during an epoch when, in fact, the basal insulin medicament regimen specifies that a basal insulin injection event must occur. Thus, the basal injection should be taken according to the prescribed basal insulin medicament regimen 208. According to the above use case, this epoch would not have a basal adherence categorization for failure to find a fasting event. In some such embodiments, because the basal insulin medicament dosage regimen 208 is known, a determination as to the adherence (of the glucose measurement during the epoch in question and/or the basal injection event in the epoch) based on the basal insulin medicament regimen itself and the injection event data, and thus does not require detecting the fasting period from the glucose sensor data. As another example, if the basal insulin medicament regimen is once weekly basal injection, the exemplary procedure would look for a basal injection within a seven day window even if a fasting event is not found.

In some embodiments, the prescribed insulin medicament dosage regimen 206 comprises a bolus insulin medicament dosage regimen 214 in addition to or instead of the basal insulin medicament dosage regimen 208.

In embodiments where the subject is taking more than one insulin mediation type, each respective insulin medicament injection event in the plurality of medicament records provides a respective type of insulin medicament injected into the subject from one of (i) a long acting insulin medicament and (ii) a short acting insulin medicament. Typically, the long acting insulin medicament is for a basal insulin medicament dosage regimen 208 whereas the short acting insulin medicament is for a bolus insulin medicament dosage regimen 214.

Thus, advantageously, the instant disclosure can also make use of the bolus insulin medicament injection events, when such events are available, to provide an additional type of categorized metabolic event 224 in the first data set 220. In some such embodiments, the bolus insulin medicament injection events are made use of in the following way. A plurality of meal events are identified using the plurality of autonomous glucose measurements 242 and the corresponding timestamps 244 in the second data set 240 using a meal detection algorithm. If no meal is detected, the process ends. If a meal is detected then a first classification is applied to the respective meal event. In this way, a plurality of meal events, with each respective meal event including a first classification that is one of "bolus regimen adherent" and "bolus regimen nonadherent" is acquired. Such information can then be used in the systems and methods of the present disclosure, where each meal is considered a metabolic event 224 and the classification of such meals as "bolus regimen adherent" and "bolus regimen nonadherent" is the first classification 228 of the metabolic event.

In some embodiments, a respective meal is deemed bolus regimen adherent when one or more medicament records in the plurality of medicament records indicates, on a temporal basis, a quantitative basis and a type of insulin medicament basis, adherence with the bolus insulin medicament dosage regimen 214 during the respective meal. In some embodiments, a respective meal is deemed bolus regimen nonadherent when the plurality of medicament records fails to indicate adherence, on a temporal basis, a quantitative basis, and a type of insulin medicament basis, with the standing bolus insulin medicament dosage regimen during the respective meal. For instance, consider the case where the standing bolus insulin medicament dosage regimen specifies that dosage A of insulin medicament B is to be taken up 30 minutes before a respective meal, or up to 15 minutes after the meal, and that a certain meal that occurred at 7:00 AM on Tuesday, May 17. It will be appreciated that dosage A may be a function of the anticipated size or type of meal. What is sought in the medicament records is evidence that the subject took dosage A of insulin medicament B in the 30 minutes leading up to 7:00 AM on Tuesday, May 17 (and not more or less of the prescribed dosage) or 15 minutes after the meal. If the subject took the prescribed dosage A of the insulin medicament B during the 30 minutes leading up to the respective meal, or within 15 minutes after the meal, the respective meal (and/or the bolus administration(s) and/or the glucose measurements during this time) is deemed bolus regimen adherent. If the subject did not take the prescribed dosage A of the insulin medicament B during the 30 minutes leading up to the respective meal or within 15 minutes after the meal (or took more than the prescribed dosage A of the insulin medicament B during this period), the respective meal (and/or the bolus administration and/or the glucose measurements during this time) is deemed bolus regimen nonadherent. The time periods in this example are exemplary. In other embodiments the time is shorter or longer (e.g., between 15 minutes to 2 hours prior to the meal and/or is dependent upon the type of insulin medicament prescribed). In other cases the standing bolus insulin medicament dosage regimen specifies that a dosage of insulin is to be taken in a time period following the meal, e.g., 30 minutes or less, 15 minutes or less, 5 minutes or less. In other cases the standing bolus insulin medicament dosage regimen specifies that a dosage of insulin is to be taken in a first predetermined time period before the meal, (e.g., 30 minutes or less, 15 minutes or less, 5 minutes or less), and/or a second predetermined time period after the meal (e.g., 30 minutes or less, 15 minutes or less, 5 minutes or less), where the first predetermined time period is the same or different than the second predetermined time period.

In some embodiments, a plurality of feed-forward events are acquired and used to help classify metabolic events. In some embodiments, each respective feed-forward event represents an instance where the subject has indicated they are having or are about to have a meal. In such embodiments, the plurality of meal events determined using the autonomous glucose measurements 242 are verified against the plurality of feed-forward events by either removing any respective meal event in the plurality of meal events that fails to temporally match any feed-forward event in the plurality of feed-forward events.

In some embodiments, the bolus insulin medicament dosage regimen 214 specifies that the short acting insulin medicament is to be taken up to a predetermined amount of time prior to or after a meal. In some such embodiments, a respective meal is deemed bolus regimen nonadherent when there is no insulin medicament record of the short acting insulin medicament type having an electronic timestamp up to the predetermined amount of time prior to or after the respective meal. In some such embodiments, the predetermined amount of time is thirty minutes or less, twenty minutes or less, or fifteen minutes or less.

In some embodiments, the long acting insulin medicament consists of a single insulin medicament having a duration of action that is between 12 and 24 hours or a mixture of insulin medicaments that collectively have a duration of action that is between 12 and 24 hours. Examples of such long acting insulin medicaments include, but are not limited to Insulin Degludec (developed by NOVO NORDISK under the brand name Tresiba), NPH (Schmid, 2007, "New options in insulin therapy. J Pediatria (Rio J). 83(Suppl 5):S146-S155), Glargine (LANTUS, March 2, 2007, insulin glargine [rDNA origin] injection, [prescribing information], Bridgewater, New Jersey: Sanofi-Aventis), and Detemir (Plank et al., 2005, "A double-blind, randomized, dose-response study investigating the pharmacodynamic and pharmacokinetic properties of the long-acting insulin analog detemir," Diabetes Care 28:1107-1112).

In some embodiments, the short acting insulin medicament consists of a single insulin medicament having a duration of action that is between three to eight hours or a mixture of insulin medicaments that collectively have a duration of action that is between three to eight hours. Examples of such short acting insulin medicaments include, but are not limited, to Lispro (HUMALOG, May 18, 2001, insulin lispro [rDNA origin] injection, [prescribing information], Indianapolis, Indiana: Eli Lilly and Company), Aspart (NOVOLOG, July 2011, insulin aspart [rDNA origin] injection, [prescribing information], Princeton, New Jersey, NOVO NORDISK Inc., July, 2011), Glulisine (Helms Kelley, 2009, "Insulin glulisine: an evaluation of its pharmacodynamic properties and clinical application," Ann Pharmacother 43:658-668), and Regular (Gerich, 2002, "Novel insulins: expanding options in diabetes management," Am J Med. 113:308-316).

In some embodiments, the identification of the plurality of meal events from the autonomous glucose measurements 242 in the second data set 240 is performed by computing: (i) a first model comprising a backward difference estimate of glucose rate of change using the plurality of autonomous glucose measurements, (ii) a second model comprising a backward difference estimate of glucose rate of change based on Kalman filtered estimates of glucose using the plurality of autonomous glucose measurements, (iii) a third model comprising a Kalman filtered estimate of glucose and Kalman filtered estimate of rate of change (ROC) of glucose based on the plurality of autonomous glucose measurements, and/or (iv) a fourth model comprising a Kalman filtered estimate of rate of change of ROC of glucose based on the plurality of autonomous glucose measurements. In some such embodiments, the first model, the second model, the third model and the fourth model are each computed across the plurality of autonomous glucose measurements and each respective meal event in the plurality of meal events is identified at an instance where at least three of the four models indicate a meal event. For further disclosure on such meal event detection, see Dassau et al., 2008, "Detection of a Meal Using Continuous Glucose Monitoring," Diabetes Care 31, pp. 295-300. See also, Cameron et al., 2009, "Probabilistic Evolving Meal Detection and Estimation of Meal Total Glucose Appearance," Journal of Diabetes Science and Technology 3(5), pp. 1022-1030.

## REFERENCES CITED AND ALTERNATIVE EMBODIMENTS

The present invention can be implemented as a computer program product that comprises a computer program mechanism embedded in a non-transitory computer readable storage medium. For instance, the computer program product could contain the program modules shown in any combination of Figures 1, 2, or 3 and/or described in Figure 4. These program modules can be stored on a CD-ROM, DVD, magnetic disk storage product, or any other non-transitory computer readable data or program storage product.

The specific embodiments described herein are offered by way of example only. The embodiments were chosen and described in order to best explain the principles of the invention and its practical applications, to thereby enable others skilled in the art to best utilize the invention and various embodiments with various modifications as are suited to the particular use contemplated. The invention is to be limited only by the terms of the appended claims.

## Claims

1. A device (250) for monitoring adherence to a prescribed insulin medicament dosage regimen (206) for a subject over time, wherein the device comprises one or more processors (274) and a memory (192/290), the memory storing instructions that, when executed by the one or more processors, perform a method of:

- obtaining a first data set (220), the first data set comprising a plurality of metabolic events the subject engaged in, wherein each respective metabolic event (224) in the plurality of metabolic events comprises (i) a timestamp (226) of the respective metabolic event and (ii) a first classification (228) that is one of insulin regimen adherent and insulin regimen nonadherent,
- classifying each respective metabolic event in the plurality of metabolic events, using a second classification, based upon the timestamp of the respective metabolic event, wherein the second classification is **characterized by** a temporal periodicity and includes a plurality of periodic elements,

binning each respective metabolic event in the plurality of metabolic events on the basis of the second classification thereby obtaining a plurality of subsets (229) of the plurality of metabolic events, wherein each respective subset (231) of the plurality of metabolic events in the plurality of subsets is for a different periodic element (233) in the plurality of periodic elements, and communicating, for each respective subset in the plurality of subsets, a respective representation of adherence (235) to the prescribed insulin medicament dosage regimen, the respective representation of adherence collectively based upon the first classification (228) of metabolic events in the respective subset, thereby monitoring adherence to the prescribed insulin medicament dosage regimen for the subject over time,

**characterized in that** the method comprises the further steps of:

- obtaining a second data set comprising a plurality of autonomous glucose measurements of the subject and, for each respective autonomous glucose measurement in the plurality of autonomous glucose measurements, a timestamp representing when the respective measurement was made,
- obtaining a third data set from one or more insulin pens used by the subject to apply the insulin medicament dosage regimen, the third data set comprises a plurality of insulin medicament records, each insulin medicament record in the plurality of medicament records comprising:

(i) a respective insulin medicament injection event including an amount of insulin medicament injected into the subject using a respective insulin pen in the one or more insulin pens, and
(ii) a corresponding electronic timestamp that is automatically generated by the respective insulin pen upon occurrence of the respective insulin medicament injection event,

- identifying the plurality of metabolic events using the plurality of autonomous glucose measurements of the subject and the respective timestamps in the second data set,
- applying a first characterization to each respective metabolic event in the plurality of metabolic events, wherein the first characterization is one of insulin regimen adherent and insulin regimen nonadherent,

wherein:

(i) a respective metabolic event is deemed basal regimen adherent when the second data set includes one or more medicament records that establish, on a temporal and quantitative basis, adherence with the insulin medicament dosage regimen during the respective metabolic event, and a respective metabolic event is deemed insulin regimen nonadherent when the second data set fails to include one or more medicament records that establish, on a temporal and quantitative basis, adherence with the insulin medicament dosage regimen,

or wherein:
(ii) the plurality of metabolic events are meal events, a respective meal event is deemed insulin regimen adherent when one or more medicament records in the plurality of medicament records indicates in the third data set, on a temporal basis, a quantitative basis, adherence with the insulin medicament dosage regimen during the respective meal, and a respective meal is deemed insulin regimen nonadherent when the plurality of medicament records in the third data set fails to indicate adherence, on a temporal basis, and a quantitative basis with the insulin medicament dosage regimen during the respective meal.

2. The device of claim 1, wherein:

each respective metabolic event in the plurality of metabolic events is within a period of time (222),
the period of time spans a plurality of weeks,
the temporal periodicity is weekly, and
each periodic element in the plurality of metabolic events is a different day in the seven days of the week.

3. The device of claim 1 or 2, wherein:

- when the insulin medicament dosage regimen is a basal insulin medicament dosage regimen (208), each respective metabolic event in the plurality of metabolic events is a fasting event.

4. The device of claim 1, wherein:

- when each respective metabolic event in the plurality of metabolic events is a meal event, each respective metabolic event in the plurality of metabolic events is within a period of time,
- the period of time spans a plurality of days, and
- the insulin medicament dosage regimen is a bolus insulin medicament dosage regimen (214).

5. The device of claim 4, wherein:

- the temporal periodicity is daily, and
- each periodic element in the plurality of periodic elements is a different one of "breakfast," "lunch," and "dinner."

6. The device of claim 4, wherein:

- the temporal periodicity is weekly, and
- each periodic element in the plurality of periodic elements represents a different meal in a set of 21 calendared weekly meals.

7. The device of any one of claims 1-6, wherein the respective representation of adherence for each respective subset in the plurality of subsets is collectively represented as a continuous two-dimensional spiral timeline comprising a plurality of revolutions by the communicating, wherein:

- the spiral timeline comprises a plurality of radial sectors,
- each revolution in the plurality of revolutions represents a period of the temporal periodicity, and
- each respective radial sector in the plurality of radial sectors is uniquely assigned a corresponding subset in the plurality of subsets.

8. The device of claim 7, the method further comprising computing a plurality of adherence values, wherein:

- each respective adherence value (232) in the plurality of adherence values represents a corresponding time window (234) in a plurality of time windows,
- each respective time window in the plurality of time windows is of a same first fixed duration,
- each respective adherence value in the plurality of adherence values is computed by dividing a number of insulin regimen adherent metabolic events by a total number of metabolic events in the plurality of metabolic events that have timestamps in the time window corresponding to the respective adherence value, and
- each respective adherence value in the plurality of adherence values is assigned to a respective radial sector in the plurality of radial sectors based upon a time period represented by the respective adherence value thereby forming, for each respective subset in the plurality of subsets, the respective representation of adherence with the prescribed insulin medicament dosage regimen.

9. The device of claim 8, wherein each respective adherence value in the two-dimensional spiral timeline is color coded as a function of an absolute value of the respective adherence value.

10. The device of any one of claims 7-9, wherein the continuous two-dimensional spiral is an Archimedean spiral or a logarithmic spiral.

11. The device of any one of claims 1-10, wherein the device includes a display (282) and the communicating includes presenting each respective representation of adherence with the prescribed insulin medicament dosage regimen on the display.

12. The device of any one of claims 1-11, wherein the device is a mobile device.

13. The device of any one of claims 1-12, the method further comprising:

- classifying each respective autonomous glucose measurement in the plurality of autonomous glucose measurements, using the second classification, based upon the timestamp of the respective autonomous glucose measurement; and wherein
- the communicating further communicates, for each respective subset in the plurality of subsets, those values of autonomous glucose measurements in the plurality of autonomous glucose measurements that have been classified into the same periodic element in the plurality of periodic elements that the respective subset represents.

14. The device of claim 13, the device further comprising a wireless receiver (284), and wherein the second data set is obtained wirelessly from a glucose sensor affixed to the subject.

15. A method of monitoring adherence to a prescribed insulin regimen for a subject, the method comprising:

- obtaining a first data set, the first data set comprising a plurality of metabolic events the subject engaged in, wherein each respective metabolic event in the plurality of metabolic events comprises (i) a timestamp of the respective metabolic event and (ii) a first classification that is one of insulin regimen adherent and insulin regimen nonadherent;
- classifying each respective metabolic event in the plurality of metabolic events, using a second classification, based upon the timestamp of the respective metabolic event, wherein the second classification is **characterized by** a temporal periodicity and includes a plurality of periodic elements;
- binning each respective metabolic event in the plurality of metabolic events on the basis of the second classification thereby obtaining a plurality of subsets of the plurality of metabolic events, wherein each respective subset of the plurality of metabolic events in the plurality of subsets is for a different periodic element in the plurality of periodic elements; and
- communicating, for each respective subset in the plurality of subsets, a respective representation of adherence to the prescribed insulin medicament dosage regimen, the respective representation of adherence collectively based upon the first classification of metabolic events in the respective subset, thereby monitoring adherence to the prescribed insulin medicament dosage regimen for the subject over time,

the method comprising the further steps of:

- obtaining a second data set comprising a plurality of autonomous glucose measurements of the subject and, for each respective autonomous glucose measurement in the plurality of autonomous glucose measurements, a timestamp representing when the respective measurement was made,
- obtaining a third data set from one or more insulin pens used by the subject to apply the insulin medicament dosage regimen, the third data set comprises a plurality of insulin medicament records, each insulin medicament record in the plurality of medicament records comprising: (i) a respective insulin medicament injection event including an amount of insulin medicament injected into the subject using a respective insulin pen in the one or more insulin pens and (ii) a corresponding electronic timestamp that is automatically generated by the respective insulin pen upon occurrence of the respective insulin medicament injection event,
- identifying the plurality of metabolic events using the plurality of autonomous glucose measurements of the subject and the respective timestamps in the second data set,
- applying a first characterization to each respective metabolic event in the plurality of metabolic events, wherein the first characterization is one of insulin regimen adherent and insulin regimen nonadherent,

wherein:

(i) a respective metabolic event is deemed basal regimen adherent when the second data set includes one or more medicament records that establish, on a temporal and quantitative basis, adherence with the insulin medicament dosage regimen during the respective metabolic event, and a respective metabolic event is deemed insulin regimen nonadherent when the second data set fails to include one or more medicament records that establish, on a temporal and quantitative basis, adherence with the insulin medicament dosage regimen,

or wherein:
(ii) the plurality of metabolic events are meal events, a respective meal event is deemed insulin regimen adherent when one or more medicament records in the plurality of medicament records indicates in the third data set, on a temporal basis, a quantitative basis, adherence with the insulin medicament dosage regimen during the respective meal, and a respective meal is deemed insulin regimen nonadherent when the plurality of medicament records in the third data set fails to indicate adherence, on a temporal basis, and a quantitative basis with the insulin medicament dosage regimen during the respective meal.

**Patentansprüche**

1. Vorrichtung (250) zum Überwachen der Einhaltung eines verschriebenen Insulinmedikamenten-Dosierungsschemas (206) für eine Person im Laufe der Zeit, wobei die Vorrichtung einen oder mehrere Prozessoren (274) und einen Speicher (192/290) umfasst, wobei der Speicher Anweisungen speichert, die bei ihrer Ausführung durch den einen oder die mehreren Prozessoren ein Verfahren ausführen, zum:

- Erhalten eines ersten Datensatzes (220), wobei der erste Datensatz eine Vielzahl von Stoffwechselereignissen umfasst, an denen die Person beteiligt war, wobei jedes jeweilige Stoffwechselereignis (224) in der Vielzahl von Stoffwechselereignissen (i) einen Zeitstempel (226) des jeweiligen Stoffwechselereignisses und (ii) eine erste Klassifizierung (228) umfasst, die eine von das Insulinschema einhaltend und das Insulinschema nicht einhaltend ist,
- Klassifizieren jedes jeweiligen Stoffwechselereignisses in der Vielzahl von Stoffwechselereignissen unter Verwendung einer zweiten Klassifizierung basierend auf dem Zeitstempel des jeweiligen Stoffwechselereignisses, wobei die zweite Klassifizierung durch eine zeitliche Periodizität gekennzeichnet ist und eine Vielzahl von periodischen Elementen beinhaltet,

Einteilen jedes jeweiligen Stoffwechselereignisses in der Vielzahl von Stoffwechselereignissen basierend auf der zweiten Klassifizierung, wodurch eine Vielzahl von Untergruppen (229) der Vielzahl von Stoffwechselereignissen erhalten wird, wobei jede jeweilige Untergruppe (231) der Vielzahl von Stoffwechselereignissen in der Vielzahl von Untergruppen für ein anderes periodisches Element (233) in der Vielzahl von periodischen Elementen ist, und Kommunizieren einer jeweiligen Darstellung der Einhaltung (235) des verschriebenen Insulinmedikamenten-Dosierungsschemas für jede jeweilige Untergruppe in der Vielzahl von Untergruppen, wobei die jeweilige Darstellung der Einhaltung gemeinsam auf der ersten Klassifizierung (228) der Stoffwechselereignisse in der jeweiligen Untergruppe basiert, wodurch die Einhaltung des verschriebenen Insulinmedikamenten-Dosierungsschemas für die Person im Laufe der Zeit überwacht wird,

**gekennzeichnet dadurch, dass** das Verfahren ferner die folgenden Schritte umfasst:

- Erhalten eines zweiten Datensatzes, der eine Vielzahl von autonomen Glukosemessungen der Person und für jede jeweilige autonome Glukosemessung in der Vielzahl der autonomen Glukosemessungen einen Zeitstempel umfasst, der angibt, wann die jeweilige Messung durchgeführt wurde,
- Erhalten eines dritten Datensatzes von einem oder mehreren Insulin-Pens, die von der Person verwendet werden, um das Insulinmedikamenten-Dosierungsschema anzuwenden, wobei der dritte Datensatz eine Vielzahl von Insulinmedikamenten-Datensätzen umfasst, wobei jeder Insulinmedikamenten-Datensatz in der Vielzahl von Medikamenten-Datensätzen umfasst:

(i) ein jeweiliges Insulinmedikament-Injektionsereignis, einschließlich einer Menge des Insulinmedikaments, das der Person unter Verwendung eines jeweiligen Insulin-Pens in dem einen oder den mehreren Insulin-Pens injiziert wird, und
(ii) einen entsprechenden elektronischen Zeitstempel, der automatisch von dem jeweiligen Insulin-Pen bei Auftreten des jeweiligen Insulinmedikamenten-Injektionsereignisses erzeugt wird,

- Identifizieren der Vielzahl von Stoffwechselereignissen unter Verwendung der Vielzahl von autonomen Glukosemessungen der Person und der jeweiligen Zeitstempel im zweiten Datensatz,
- Anwendung einer ersten Charakterisierung auf jedes jeweilige Stoffwechselereignis in der Vielzahl der Stoffwechselereignisse, wobei die erste Charakterisierung eine von das Insulinschema einhaltenden und das Insulinschema nicht einhaltenden ist,

wobei:

(i) ein jeweiliges Stoffwechselereignis als Basalschema einhaltend angesehen wird, wenn der zweite Datensatz einen oder mehrere Medikamenten-Datensätze beinhaltet, die auf einer zeitlichen und quantitativen Basis die Einhaltung des Insulinmedikamenten-Dosierungsschemas während des jeweiligen Stoffwechselereignisses belegen, und ein jeweiliges Stoffwechselereignis als nicht das Insulinmedikamenten-Dosierungsschema einhaltend angesehen wird, wenn der zweite Datensatz nicht einen oder mehrere Medikamenten-Datensätze beinhaltet, die auf einer zeitlichen und quantitativen Basis die Einhaltung des Insulinmedikamenten-Dosierungsschemas belegen,

oder wobei:
(ii) die Vielzahl von Stoffwechselereignissen Mahlzeitereignisse sind, wobei ein jeweiliges Mahlzeitereignis als Insulinschema einhaltend angesehen wird, wenn ein oder mehrere Medikamenten-Datensätze in der Vielzahl von Medikamenten-Datensätzen in dem dritten Datensatz auf einer zeitlichen Basis und einer quantitativen Basis die Einhaltung des Insulinmedikamenten-Dosierungsschemas während der jeweiligen Mahlzeit anzeigt, und eine entsprechende Mahlzeit als das Insulinmedikamenten-Dosierungsschema nicht einhaltend angesehen wird, wenn die Vielzahl der Medikamenten-Datensätze in dem dritten Datensatz auf einer zeitlichen Basis und einer quantitativen Basis nicht die Einhaltung des Insulinmedikamenten-Dosierungsschemas während der entsprechenden Mahlzeit anzeigt.

2. Vorrichtung nach Anspruch 1, wobei:

jedes jeweilige Stoffwechselereignis in der Vielzahl der Stoffwechselereignisse innerhalb eines Zeitraums (222) liegt, der Zeitraum sich über mehrere Wochen erstreckt, die zeitliche Periodizität wöchentlich ist, und jedes periodische Element in der Vielzahl der Stoffwechselereignisse ein anderer Tag in den sieben Tagen der Woche ist.

3. Vorrichtung nach Anspruch 1 oder 2, wobei:

- wenn es sich bei dem Insulinmedikamenten-Dosierungsschema um ein Basalinsulinmedikamenten-Dosierungsschema (208) handelt, jedes jeweilige Stoffwechselereignis in der Vielzahl der Stoffwechselereignisse ein Fastenereignis ist.

4. Vorrichtung nach Anspruch 1, wobei:

- wenn jedes jeweilige Stoffwechselereignis in der Vielzahl der Stoffwechselereignisse ein Mahlzeitereignis ist, jedes jeweilige Stoffwechselereignis in der Vielzahl der Stoffwechselereignisse innerhalb eines Zeitraums liegt,
- der Zeitraum sich über mehrere Tage erstreckt, und
- das Insulinmedikamenten-Dosierungsschema ein Bolus-Insulinmedikamenten-Dosierungsschema (214) ist.

5. Vorrichtung nach Anspruch 4, wobei:

- die zeitliche Periodizität täglich ist, und
- jedes periodische Element in der Vielzahl der periodischen Elemente ein anderes von "Frühstück", "Mittagessen" und "Abendessen" ist.

6. Vorrichtung nach Anspruch 4, wobei:

- die zeitliche Periodizität wöchentlich ist, und
- jedes periodische Element in der Vielzahl der periodischen Elemente eine andere Mahlzeit in einem Satz von 21 kalendarischen wöchentlichen Mahlzeiten darstellt.

7. Vorrichtung nach einem der Ansprüche 1 bis 6, wobei die jeweilige Darstellung der Einhaltung für jede jeweilige Untergruppe in der Vielzahl von Untergruppen kollektiv als eine kontinuierliche zweidimensionale spiralförmige Zeitleiste dargestellt ist, die eine Vielzahl von Umdrehungen durch das Kommunizieren umfasst, wobei:

- die spiralförmige Zeitleiste eine Vielzahl von radialen Sektoren umfasst,
- jede Umdrehung in der Vielzahl der Umdrehungen eine Periode der zeitlichen Periodizität darstellt, und
- jeder jeweilige radiale Sektor in der Vielzahl der radialen Sektoren eindeutig einer entsprechenden Untergruppe in der Vielzahl der Untergruppen zugeordnet ist.

8. Vorrichtung nach Anspruch 7, wobei das Verfahren ferner das Berechnen einer Vielzahl von Einhaltungswerten umfasst, wobei:

- jeder jeweilige Einhaltungswert (232) in der Vielzahl von Einhaltungswerten ein entsprechendes Zeitfenster (234) in einer Vielzahl von Zeitfenstern darstellt,
- jedes jeweilige Zeitfenster in der Vielzahl von Zeitfenstern die gleiche erste feste Dauer hat,
- jeder jeweilige Einhaltungswert in der Vielzahl von Einhaltungswerten berechnet wird, indem eine Anzahl von das Insulinschema einhaltenden Stoffwechselereignissen durch eine Gesamtzahl von Stoffwechselereignissen in der Vielzahl von Stoffwechselereignissen geteilt wird, die Zeitstempel in dem Zeitfenster aufweisen, das dem jeweiligen Einhaltungswert entspricht, und
- jeder jeweilige Einhaltungswert in der Vielzahl von Einhaltungswerten einem jeweiligen radialen Sektor in der Vielzahl von radialen Sektoren basierend auf einem durch den jeweiligen Einhaltungswert dargestellten Zeitraum zugewiesen wird, wodurch für jede jeweilige Untergruppe in der Vielzahl von Untergruppen die jeweilige Darstellung der Einhaltung des verschriebenen Insulinmedikamenten-Dosierungsschemas gebildet wird.

9. Vorrichtung nach Anspruch 8, wobei jeder jeweilige Einhaltungswert in der zweidimensionalen spiralförmigen Zeitleiste als Funktion eines absoluten Werts des jeweiligen Einhaltungswerts farbcodiert ist.

10. Vorrichtung nach einem der Ansprüche 7 bis 9, wobei die kontinuierliche zweidimensionale Spirale eine archimedische Spirale oder eine logarithmische Spirale ist.

11. Vorrichtung nach einem der Ansprüche 1 bis 10, wobei die Vorrichtung eine Anzeige (282) beinhaltet und die Kommunikation die Darstellung jeder jeweiligen Darstellung der Einhaltung des verschriebenen Insulinmedikamenten-Dosierungsschemas auf der Anzeige beinhaltet.

12. Vorrichtung nach einem der Ansprüche 1 bis 11, wobei die Vorrichtung eine mobile Vorrichtung ist.

13. Vorrichtung nach einem der Ansprüche 1-12, das Verfahren ferner umfassend:

- Klassifizieren jeder jeweiligen autonomen Glukosemessung in der Vielzahl der autonomen Glukosemessungen unter Verwendung der zweiten Klassifizierung basierend auf dem Zeitstempel der jeweiligen autonomen Glukosemessung, und wobei
- die Kommunikation ferner für jede jeweilige Untergruppe in der Vielzahl von Untergruppen diejenigen Werte von autonomen Glukosemessungen in der Vielzahl von autonomen Glukosemessungen kommuniziert, die in das gleiche periodische Element in der Vielzahl von periodischen Elementen klassifiziert worden sind, das die jeweilige Untergruppe darstellt.

14. Vorrichtung nach Anspruch 13, wobei die Vorrichtung ferner einen drahtlosen Empfänger (284) umfasst, und wobei der zweite Datensatz drahtlos von einem an der Person befestigten Glukosesensor erhalten wird.

15. Verfahren zur Überwachung der Einhaltung eines verschriebenen Insulinschemas für eine Person, das Verfahren umfassend:

- Erhalten eines ersten Datensatzes, wobei der erste Datensatz eine Vielzahl von Stoffwechselereignissen umfasst, an denen die Person beteiligt war, wobei jedes jeweilige Stoffwechselereignis in der Vielzahl von Stoffwechselereignissen (i) einen Zeitstempel des jeweiligen Stoffwechselereignisses und (ii) eine erste Klassifizierung umfasst, die eine von das Insulinschema einhaltend und das Insulinschema nicht einhaltend ist,
- Klassifizieren jedes jeweiligen Stoffwechselereignisses in der Vielzahl von Stoffwechselereignissen unter Verwendung einer zweiten Klassifizierung basierend auf dem Zeitstempel des jeweiligen Stoffwechselereignisses, wobei die zweite Klassifizierung durch eine zeitliche Periodizität gekennzeichnet ist und eine Vielzahl von periodischen Elementen beinhaltet,
- Einteilen jedes jeweiligen Stoffwechselereignisses in der Vielzahl von Stoffwechselereignissen basierend auf der zweiten Klassifizierung, wodurch eine Vielzahl von Untergruppen der Vielzahl von Stoffwechselereignissen erhalten wird, wobei jede jeweilige Untergruppe der Vielzahl von Stoffwechselereignissen in der Vielzahl von Untergruppen für ein anderes periodisches Element in der Vielzahl von periodischen Elementen ist, und
- Kommunizieren einer jeweiligen Darstellung der Einhaltung des verschriebenen Insulinmedikamenten-Dosierungsschemas für jede jeweilige Untergruppe in der Vielzahl von Untergruppen, wobei die jeweilige Darstellung der Einhaltung gemeinsam auf der ersten Klassifizierung der Stoffwechselereignisse in der jeweiligen Untergruppe basiert, wodurch die Einhaltung des verschriebenen Insulinmedikamenten-Dosierungsschemas für die Person über die Zeit überwacht wird,

das Verfahren umfassend die weiteren Schritte:

- Erhalten eines zweiten Datensatzes, der eine Vielzahl von autonomen Glukosemessungen der Person und für jede jeweilige autonome Glukosemessung in der Vielzahl der autonomen Glukosemessungen einen Zeitstempel umfasst, der angibt, wann die jeweilige Messung durchgeführt wurde,
- Erhalten eines dritten Datensatzes von einem oder mehreren Insulin-Pens, die von der Person zur Anwendung des Insulinmedikamenten-Dosierungsschemas verwendet werden, wobei der dritte Datensatz eine Vielzahl von Insulinmedikamenten-Datensätzen umfasst und jeder Insulinmedikamenten-Datensatz in der Vielzahl von Medikamenten-Datensätzen umfasst: (i) ein entsprechendes Insulinmedikamenten-Injektionsereignis einschließlich einer Insulinmedikamentenmenge, die der Person unter Verwendung eines entsprechenden Insulin-Pens von dem einen oder den mehreren Insulin-Pens injiziert wurde, und (ii) einen entsprechenden elektronischen Zeitstempel, der beim Auftreten des entsprechenden Insulinmedikamenten-Injektionsereignisses durch den entsprechenden Insulin-Pen automatisch erzeugt wird,
- Identifizieren der Vielzahl von Stoffwechselereignissen unter Verwendung der Vielzahl von autonomen Glukosemessungen der Person und der jeweiligen Zeitstempel im zweiten Datensatz,
- Anwendung einer ersten Charakterisierung auf jedes jeweilige Stoffwechselereignis in der Vielzahl der Stoffwechselereignisse, wobei die erste Charakterisierung eine von das Insulinschema einhaltenden und das Insulinschema nicht einhaltenden ist,

wobei:

(i) ein jeweiliges Stoffwechselereignis als Basalschema einhaltend angesehen wird, wenn der zweite Datensatz einen oder mehrere Medikamenten-Datensätze beinhaltet, die auf einer zeitlichen und quantitativen Basis die Einhaltung des Insulinmedikamenten-Dosierungsschemas während des jeweiligen Stoffwechselereignisses belegen, und ein jeweiliges Stoffwechselereignis als nicht das Insulinmedikamenten-Dosierungsschema einhaltend angesehen wird, wenn der zweite Datensatz nicht einen oder mehrere Medikamenten-Datensätze beinhaltet, die auf einer zeitlichen und quantitativen Basis die Einhaltung des Insulinmedikamenten-Dosierungsschemas belegen,

oder wobei:
(ii) die Vielzahl von Stoffwechselereignissen Mahlzeitereignisse sind, wobei ein jeweiliges Mahlzeitereignis als Insulinschema einhaltend angesehen wird, wenn ein oder mehrere Medikamenten-Datensätze in der Vielzahl von Medikamenten-Datensätzen in dem dritten Datensatz auf einer zeitlichen Basis und einer quantitativen Basis die Einhaltung des Insulinmedikamenten-Dosierungsschemas während der jeweiligen Mahlzeit anzeigt, und eine entsprechende Mahlzeit als das Insulinmedikamenten-Dosierungsschema nicht einhaltend angesehen wird, wenn die Vielzahl der Medikamenten-Datensätze in dem dritten Datensatz auf einer zeitlichen Basis und einer quantitativen Basis nicht die Einhaltung des Insulinmedikamenten-Dosierungsschemas während der entsprechenden Mahlzeit anzeigt.

## Revendications

1. Dispositif (250) destiné à surveiller le respect d'un régime posologique médicamenteux insulinique prescrit (206) pour un sujet au cours du temps, ledit dispositif comprenant un ou plusieurs processeurs (274) et une mémoire (192/290), la mémoire stockant des instructions qui, lorsqu'elles sont exécutées par le ou les processeurs, réalisent un procédé visant à :

- obtenir un premier ensemble de données (220), le premier ensemble de données comprenant une pluralité d'événements métaboliques entrepris par le sujet, chaque événement métabolique (224) respectif de la pluralité d'événements métaboliques comprenant (i) une estampille temporelle (226) de l'événement métabolique respectif et (ii) une première classification (228) indicative du respect du régime d'insuline ou du non-respect du régime d'insuline,
- classifier chaque événement métabolique respectif de la pluralité d'événements métaboliques à l'aide d'une deuxième classification en fonction de l'estampille temporelle de l'événement métabolique respectif, ladite deuxième classification étant **caractérisée par** une périodicité temporelle et comportant une pluralité d'éléments périodiques,

grouper chaque événement métabolique respectif de la pluralité d'événements métaboliques compte tenu de la deuxième classification, pour ainsi obtenir une pluralité de sous-ensembles (229) de la pluralité d'événements métaboliques, chaque sous-ensemble respectif (231) de la pluralité d'événements métaboliques au sein de la pluralité de sous-ensembles étant relatif à un élément périodique différent (233) de la pluralité d'éléments périodiques, et communiquer, pour chaque sous-ensemble respectif de la pluralité de sous-ensembles, une représentation respective du respect (235) du régime posologique médicamenteux insulinique prescrit, la représentation respective du respect reposant collectivement sur la première classification (228) des événements métaboliques au sein du sous-ensemble respectif, pour ainsi surveiller le respect du régime posologique médicamenteux insulinique prescrit pour le sujet au cours du temps,

**caractérisé en ce que** le procédé comprend les étapes supplémentaires consistant à :

- obtenir un deuxième ensemble de données comprenant une pluralité de mesures autonomes de glycémie du sujet et, pour chaque mesure autonome de glycémie respective de la pluralité de mesures autonomes de glycémie, une estampille temporelle représentant le moment auquel la mesure respective a été effectuée,
- obtenir un troisième ensemble de données en provenance d'un ou plusieurs stylos à insuline utilisés par le sujet pour appliquer le régime posologique médicamenteux insulinique, le troisième ensemble de données comprenant une pluralité d'enregistrements de médicament insulinique, chaque enregistrement de médicament insulinique de la pluralité d'enregistrements de médicament comprenant :

(i) un événement d'injection de médicament insulinique respectif comprenant une quantité de médicament insulinique injectée au sujet à l'aide d'un stylo à insuline respectif parmi le ou les stylos à insuline, et
(ii) une estampille temporelle électronique correspondante qui est générée automatiquement par le stylo à insuline respectif lors de la survenue de l'événement d'injection de médicament insulinique respectif,

- identifier la pluralité d'événements métaboliques à l'aide de la pluralité de mesures autonomes de glycémie du sujet et des estampilles temporelles respectives du deuxième ensemble de données,
- appliquer une première caractérisation à chaque événement métabolique respectif de la pluralité d'événements métaboliques, ladite première caractérisation étant indicative du respect du régime d'insuline ou du non-respect du régime d'insuline,

étant entendu que :

(i) un événement métabolique respectif est considéré respecter le régime basal lorsque le deuxième ensemble de données comprend un ou plusieurs enregistrements de médicament qui établissent, dans un cadre temporel et quantitatif, le respect du régime posologique médicamenteux insulinique pendant l'événement métabolique respectif, et un événement métabolique respectif est considéré ne pas respecter le régime d'insuline lorsque le deuxième ensemble de données ne comprend pas le ou les enregistrements de médicament qui établissent, dans un cadre temporel et quantitatif, le respect du régime posologique médicamenteux insulinique,

ou bien :

(ii) la pluralité d'événements métaboliques sont des événements de repas, un événement de repas respectif est considéré respecter le régime d'insuline lorsqu'un ou plusieurs enregistrements de médicament de la pluralité d'enregistrements de médicament indiquent dans le troisième ensemble de données, dans un cadre temporel, un cadre quantitatif, le respect du régime posologique médicamenteux insulinique pendant le repas respectif, et un repas respectif est considéré ne pas respecter le régime d'insuline lorsque la pluralité d'enregistrements de médicament du troisième ensemble de données n'est pas indicatif du respect, dans un cadre temporel et un cadre quantitatif, du régime posologique médicamenteux insulinique pendant le repas respectif.

2. Dispositif selon la revendication 1, dans lequel :

chaque événement métabolique respectif de la pluralité d'événements métaboliques s'inscrit dans une certaine période (222), la période s'étend sur plusieurs semaines,

la périodicité temporelle est hebdomadaire, et chaque élément périodique de la pluralité d'événements métaboliques est un jour différent parmi les sept jours de la semaine.

3. Dispositif selon la revendication 1 ou 2, dans lequel :

- lorsque le régime posologique médicamenteux insulinique est un régime posologique médicamenteux insulinique basal (208), chaque événement métabolique de la pluralité d'événements métaboliques est un événement de jeûne.

4. Dispositif selon la revendication 1, dans lequel :

- lorsque chaque événement métabolique respectif de la pluralité d'événements métaboliques est un événement de repas, chaque événement métabolique respectif de la pluralité d'événements métaboliques se situe dans une certaine période,
- la période s'étend sur une pluralité de jours, et
- le régime posologique médicamenteux insulinique est un régime posologique médicamenteux insulinique en bolus (214).

5. Dispositif selon la revendication 4, dans lequel :

- la périodicité temporelle est quotidienne, et
- chaque élément périodique de la pluralité d'éléments périodiques est un élément différent parmi le petit-déjeuner, le déjeuner et le dîner.

6. Dispositif selon la revendication 4, dans lequel :

- la périodicité temporelle est hebdomadaire, et
- chaque élément périodique de la pluralité d'éléments périodiques représente un repas différent dans un ensemble de 21 repas hebdomadaires planifiés.

7. Dispositif selon l'une quelconque des revendications 1 à 6, dans lequel la représentation respective du respect pour chaque sous-ensemble respectif de la pluralité de sous-ensembles est représentée collectivement sous la forme d'une ligne de temps en spirale bidimensionnelle continue comprenant une pluralité de révolutions, dans le cadre de l'opération de communication, étant entendu que :

- la ligne temporelle en spirale comprend une pluralité de secteurs radiaux,
- chaque révolution de la pluralité de révolutions représente une période de la périodicité temporelle, et
- chaque secteur radial respectif de la pluralité de secteurs radiaux se voit attribuer de manière unique un sous-ensemble correspondant de la pluralité de sous-ensembles.

8. Dispositif selon la revendication 7, le procédé comprenant en outre le calcul d'une pluralité de valeurs de respect, étant entendu que :

- chaque valeur de respect respective (232) de la pluralité de valeurs de respect représente une fenêtre temporelle (234) correspondante parmi une pluralité de fenêtres temporelles,
- chaque fenêtre temporelle respective de la pluralité de fenêtres temporelles est d'une même première durée fixe,
- chaque valeur de respect respective de la pluralité de valeurs de respect est calculée en divisant le nombre d'événements métaboliques respectant le régime d'insuline par le nombre total d'événements métaboliques de la pluralité d'événements métaboliques qui ont des estampilles temporelles situées dans la fenêtre temporelle correspondant à la valeur de respect respective, et
- chaque valeur de respect respective de la pluralité de valeurs de respect est attribuée à un secteur radial respectif de la pluralité de secteurs radiaux compte tenu d'une période de temps représentée par la valeur de respect respective, pour ainsi former, pour chaque sous-ensemble respectif de la pluralité de sous-ensembles, la représentation respective du respect du régime posologique médicamenteux insulinique prescrit.

9. Dispositif selon la revendication 8, dans lequel chaque valeur de respect respective dans la ligne de temps en spirale bidimensionnelle fait l'objet d'un code couleur en fonction d'une valeur absolue de la valeur de respect respective.

10. Dispositif selon l'une quelconque des revendications 7 à 9, dans lequel la spirale bidimensionnelle continue est une spirale d'Archimède ou une spirale logarithmique.

11. Dispositif selon l'une quelconque des revendications 1 à 10, ledit dispositif comprenant un écran (282) et la communication comprenant la présentation de chaque représentation respective du respect du régime posologique médicamenteux insulinique prescrit sur l'écran.

12. Dispositif selon l'une quelconque des revendications 1 à 11, ledit dispositif étant un dispositif mobile.

13. Dispositif selon l'une quelconque des revendications 1 à 12, le procédé comprenant en outre :

- la classification de chaque mesure autonome de glycémie respective de la pluralité de mesures autonomes de glycémie, à l'aide de la deuxième classification, compte tenu de l'estampille temporelle de la mesure autonome de glycémie respective ; étant entendu que
- la communication permet de communiquer en outre, pour chaque sous-ensemble respectif de la pluralité de sous-ensembles, les valeurs de mesures autonomes de glycémie de la pluralité de mesures autonomes de glycémie qui ont été classifiées dans le même élément périodique de la pluralité d'éléments périodiques que le sous-ensemble respectif représente.

14. Dispositif selon la revendication 13, le dispositif comprenant en outre un récepteur sans fil (284), et dans lequel le deuxième ensemble de données est obtenu sans fil à partir d'un capteur de glycémie fixé sur le sujet.

15. Procédé de surveillance du respect d'un régime posologique médicamenteux insulinique prescrit pour un sujet, le procédé comprenant les opérations consistant à :

- obtenir un premier ensemble de données, le premier ensemble de données comprenant une pluralité d'événements métaboliques entrepris par le sujet, chaque événement métabolique respectif de la pluralité d'événements métaboliques comprenant (i) une estampille temporelle de l'événement métabolique respectif et (ii) une première classification indicative du respect du régime d'insuline et du non-respect du régime d'insuline ;
- classifier chaque événement métabolique respectif de la pluralité d'événements métaboliques à l'aide d'une deuxième classification en fonction de l'estampille temporelle de l'événement métabolique respectif, ladite deuxième classification étant **caractérisée par** une périodicité temporelle et comportant une pluralité d'éléments périodiques ;
- grouper chaque événement métabolique respectif de la pluralité d'événements métaboliques compte tenu de la deuxième classification, pour ainsi obtenir une pluralité de sous-ensembles de la pluralité d'événements métaboliques, chaque sous-ensemble respectif de la pluralité d'événements métaboliques au sein de la pluralité de sous-ensembles étant relatif à un élément périodique différent de la pluralité d'éléments périodiques ; et
- communiquer, pour chaque sous-ensemble respectif de la pluralité de sous-ensembles, une représentation respective du respect du régime posologique médicamenteux insulinique prescrit, la représentation respective du respect reposant collectivement sur la première classification des événements métaboliques au sein du sous-ensemble respectif, pour ainsi surveiller le respect du régime posologique médicamenteux insulinique prescrit pour le sujet au cours du temps,

le procédé comprenant les étapes supplémentaires consistant à :

- obtenir un <u>deuxième</u> ensemble de données comprenant une pluralité de mesures autonomes de glycémie du sujet et, pour chaque mesure autonome de glycémie respective de la pluralité de mesures autonomes de glycémie, une estampille temporelle représentant le moment auquel la mesure respective a été effectuée,
- obtenir un <u>troisième</u> ensemble de données en provenance d'un ou plusieurs stylos à insuline utilisés par le sujet pour appliquer le régime posologique médicamenteux insulinique, le troisième ensemble de données comprenant une pluralité d'enregistrements de médicament insulinique, chaque enregistrement de médicament insulinique de la pluralité d'enregistrement de médicament comprenant : (i) un événement d'injection de médicament insulinique respectif comprenant une quantité de médicament insulinique injectée au sujet à l'aide d'un stylo à insuline respectif parmi le ou les stylos à insuline, et (ii) une estampille temporelle électronique correspondante qui est générée automatiquement par le stylo à insuline respectif lors de la survenue de l'événement d'injection de médicament insulinique respectif,
- identifier la pluralité d'événements métaboliques à l'aide de la pluralité de mesures autonomes de glycémie du sujet et des estampilles temporelles respectives du deuxième ensemble de données,
- appliquer une première caractérisation à chaque événement métabolique respectif de la pluralité d'événements métaboliques, ladite première caractérisation étant indicative du respect du régime d'insuline ou du non-respect du régime d'insuline,

étant entendu que :

(i) un événement métabolique respectif est considéré respecter le régime basal lorsque le deuxième ensemble de données comprend un ou plusieurs enregistrements de médicament qui établissent, dans un cadre temporel et quantitatif, le respect du régime posologique médicamenteux insulinique pendant l'événement métabolique respectif, et un événement métabolique respectif est considéré ne pas respecter le régime d'insuline lorsque le deuxième ensemble de données ne comprend pas le ou les enregistrements de médicament qui établissent, dans un cadre temporel et quantitatif, le respect du régime posologique médicamenteux insulinique,

ou bien :

(ii) la pluralité d'événements métaboliques sont des événements de repas, un événement de repas respectif est considéré respecter le régime d'insuline lorsqu'un ou plusieurs enregistrements de médicament de la pluralité d'enregistrements de médicament indiquent dans le troisième ensemble de données, dans un cadre temporel, un cadre quantitatif, le respect du régime posologique médicamenteux insulinique pendant le repas respectif, et un repas respectif est considéré ne pas respecter le régime d'insuline lorsque la pluralité d'enregistrements de médicament du troisième ensemble de données n'est pas indicatif du respect, dans un cadre temporel et un cadre quantitatif, du régime posologique médicamenteux insulinique pendant le repas respectif.

Regimen Adherence Monitor 250
(Monitor Device)

48

502

Regimen Adherence Assessor 200
(Adherence Device)

Communication Network(s)
106

102-1

Glucose sensor 1

102-P

Glucose sensor P

104-1

Insulin pen 1 / pump 1

104-Q

Insulin pen Q / pump Q

Fig. 1

| | |
|---|---|
| Operating system | 202 |
| Insulin regimen monitoring module | 204 |
| Prescribed insulin medicament dosage regimen | 206 |
|   Basal insulin medicament dosage regimen | 208 |
|     Dose of long acting insulin medicament 1 | 210-1 |
|     Epoch 1 | 212-1 |
|     ⋮ | |
|     Dose of long acting insulin medicament N | 210-N |
|     Epoch N | 212-N |
|   Bolus insulin medicament dosage regimen | 214 |
|     Dosage 1 | 216-1 |
|     Date / Time 1 | 218-1 |
|     ⋮ | |
|     Dosage M | 216-M |
|     Date / Time M | 218-M |
| First data set | 220 |
|   First period of time | 222 |
|   Metabolic event 1 | 224-1 |
|     Timestamp of metabolic event 1 | 226-1 |
|     Classification of metabolic event 1 | 228-1 |
|     ⋮ | 231-1 |
|   Metabolic event Q | 224-Q |
| Plurality of subsets | 229 |
|   Subset 1 | 231-1 |
|     Periodic element 1 | 233-1 |
|       Metabolic event 1-1 | 224-1-1 |
|       ⋮ | |
|       Metabolic event 1-P | 224-1-P |
|     Representation of adherence 1 | 235-1 |
|       Adherence value 1-1 | 232-1-1 |
|         Time window 1-1 | 234-1-1 |
|         ⋮ | |
|       Adherence value 1-X | 234-1-X |
|     ⋮ | |
|   Subset Q | |
| Second data set | 240 |
| • • • | |

250

192

290

Controller
288

CPU
274

Power
supply
276

User interface
278
  Display — 282
  Keyboard — 280

Network
interface
284

212

Network

Fig. 2

## 250

| Operating system | 202 |
| Insulin regimen monitoring module | 204 |
| Prescribed insulin medicament dosage regimen | 206 |
| First data set | 220 |
| Plurality of subsets | 229 |
| Optional accelerometer driver | 330 |
| Second data set | 240 |

192 — First data set

| | | |
|---|---|---|
| | Autonomous glucose measurement 1 | 242-1 |
| | Glucose measurement timestamp 1 | 244-1 |
| | ⋮ | |
| | Autonomous glucose measurement R | 242-R |
| | Glucose measurement timestamp R | 244-R |

Memory controller 368

Network 106

212

CPU 274

Power supply 276

User interface 278
Display 282
Input 280

Network interface including RF circuitry 284

364

Optional intensity sensors

Optical sensor(s) 373

Optional accelerometer 317

Optional GPS 319

Audio circuitry 372

Optional speaker 360

Optional microphone 362

Peripherals interface 370

Input/output (I/O) subsystem 366

## Fig. 3

402

Methods for monitoring adherence to a prescribed insulin medicament dosage regimen 206 for a subject over time using a device 250 are provided. A first data set 220 is obtained. The first data set comprises a plurality of metabolic events in which the subject engaged. The plurality of metabolic events are within a period of time 222. Each respective metabolic event 224 in the plurality of metabolic events comprises (i) a timestamp 226 of the respective metabolic event and (ii) a first classification 228 that is one of insulin regimen adherent and insulin regimen nonadherent.

404

The device is a mobile device

406

Classify each respective metabolic event in the plurality of metabolic events, using a second classification, based upon the timestamp of the respective metabolic event. The second classification is characterized by a temporal periodicity and includes a plurality of periodic elements.

408

Each respective metabolic event in the plurality of metabolic events is within a period of time that spans a plurality of weeks. The temporal periodicity is weekly, and each periodic element in the plurality of metabolic events is a different day in the seven days of the week.

410

Each respective metabolic event in the plurality of metabolic events is a fasting event and the insulin medicament dosage regimen is a basal insulin medicament dosage regimen.

412

Each respective metabolic event in the plurality of metabolic events is within a period of time spanning a plurality of days. Each respective metabolic event in the plurality of metabolic events is a meal event. The insulin medicament dosage regimen is a bolus insulin medicament dosage regimen.

414

The temporal periodicity is daily, and each periodic element in the plurality of periodic elements is a different one of "breakfast," "lunch," and "dinner."

416

The temporal periodicity is weekly, and each periodic element in the plurality of periodic elements represents a different meal in a set of 21 calendared weekly meals.

(A)

Fig. 4A

418

Bin each respective metabolic event in the plurality of metabolic events on the basis of the second classification thereby obtaining a plurality of subsets (229) of the plurality of metabolic events. Each respective subset (231) of the plurality of metabolic events in the plurality of subsets is for a different periodic element in the plurality of periodic elements.

420

Communicate, for each respective subset in the plurality of subsets, a respective representation of adherence (235) to the prescribed insulin medicament dosage regimen. The respective representation of adherence collectively based upon the first classification of metabolic events in the respective subset, thereby monitoring adherence to the prescribed insulin medicament dosage regimen for the subject over time.

422

The respective representation of adherence for each respective subset in the plurality of subsets is collectively represented as a continuous two-dimensional spiral timeline comprising a plurality of revolutions. The spiral timeline comprises a plurality of radial sectors. Each revolution in the plurality of revolutions represents a period of the temporal periodicity. Each respective radial sector in the plurality of radial sectors is uniquely assigned a corresponding subset in the plurality of subsets.

424

A plurality of adherence values are computed. Each respective adherence value (232) in the plurality of adherence values represents a corresponding time window (234) in a plurality of time windows. Each respective time window in the plurality of time windows is of a same first fixed duration. Each respective adherence value in the plurality of adherence values is computed by dividing a number of insulin regimen adherent metabolic events by a total number of metabolic events in the plurality of metabolic events that have timestamps in the time window corresponding to the respective adherence value. Each respective adherence value in the plurality of adherence values is assigned to a respective radial sector in the plurality of radial sectors based upon a time period represented by the respective adherence value thereby forming, for each respective subset in the plurality of subsets, the respective representation of adherence with the prescribed insulin medicament dosage regimen.

426

Each respective adherence value in the two-dimensional spiral timeline is color coded as a function of an absolute value of the respective adherence value.

B

Fig. 4B

B

420
(cont.)
422
(cont.)
428

The continuous two-dimensional spiral is an Archimedean spiral or a logarithmic spiral.

430

The device includes a display and the communicating the representation of adherence includes presenting each respective representation of adherence with the prescribed insulin medicament dosage regimen on the display.

432

The method further comprises obtaining a second data set (240). The second data set comprises a plurality of autonomous glucose measurements of the subject. For each respective autonomous glucose measurement (242) in the plurality of autonomous glucose measurements, there is a timestamp (244) representing when the respective measurement was made. Each respective autonomous glucose measurement in the plurality of autonomous glucose measurements is classified using the second classification, based upon the timestamp of the respective autonomous glucose measurement. The communicating further communicates, for each respective subset in the plurality of subsets, those values of autonomous glucose measurements in the plurality of autonomous glucose measurements that have been classified into the same periodic element in the plurality of periodic elements that the respective subset represents.

434

The device further comprises a wireless receiver. The second data set is obtained wirelessly from a glucose sensor affixed to the subject.

Fig. 4C

Fig. 5

Basal regimen: take basal dose twice per day, one in the morning and one at night time (app. 12 hours between). The characterization of a metabolic event, being a fasting event, is provided as follows:

*For every date (primary period being a day and relevant period defined by the regimen being a day):*

> *Were two basal injections detected?*
>> *If no*
>>> *Metabolic event marked out of basal adherence, B2*
>> *If yes*
>>> *Was the time between basal injections >10 and <14 hours?*
>>>> *If yes*
>>>>> *Mark metabolic event as in basal and timing adherence, B1, C1*
>>>> *If no*
>>>>> *Mark metabolic event as in basal adherence, but out of timing adherence, B1, C1.*

# Fig. 6

Temporal Periodicity →

| Periodic element 233-1 (Monday) | Periodic element 233-2 (Tuesday) | Periodic element 233-3 (Wednesday) | Periodic element 233-4 (Thursday) | Periodic element 233-5 (Friday) | Periodic element 233-6 (Saturday) | Periodic element 233-7 (Sunday) |
|---|---|---|---|---|---|---|

702-1

Metabolic events in week 1 of the first data set 220

224-1   224-2   224-3   224-4   224-5   224-6   224-7

702-2

Metabolic events in week 2 of the first data set 220

224-8   224-9   224-10   224-11   224-12   224-13   224-14

⋮

702-W

Metabolic events in week W of the first data set 220

224-(Q-6)   224-(Q-5)   224-(Q-4)   224-(Q-3)   224-(Q-2)   224-(Q-1)   224-Q

Key

▨ Insulin regimen adherent metabolic event 702

▨ Insulin regimen nonadherent metabolic event 704

Fig. 7

EP 3 479 263 B1

← Temporal Periodicity →

| Periodic element 233-1 (Breakfast) | Periodic element 233-2 (Lunch) | Periodic element 233-3 (Dinner) |
|---|---|---|

802-1

Metabolic events in day 1 of the first data set 220

224-1   224-2   224-3

802-2

Metabolic events in day 2 of the first data set 220

224-8   224-9   224-10

⋮

802-W

Metabolic events in day W of the first data set 220

224-(Q-2)   224-(Q-1)   224-Q

| Key |
|---|
| ▨ Insulin regimen adherent metabolic event 702 |
| ▧ Insulin regimen nonadherent metabolic event 704 |

Fig. 8

Fig. 9

EP 3 479 263 B1

← Temporal Periodicity →

| Periodic element 233-1 (Breakfast) | Periodic element 233-2 (Lunch) | Periodic element 233-3 (Dinner) |
|---|---|---|

802-1

| Metabolic events in day 1 of the first data set 220 |
|---|

802-2

| Metabolic events in day 2 of the first data set 220 |
|---|

224-1

224-2

224-3

224-8

224-9

224-10

⋮

802-W

| Metabolic events in day W of the first data set 220 |
|---|

224-(Q-2)

224-(Q-1)

224-Q

231-1      231-2      231-3

| Key |
|---|
| ▨ Insulin regimen adherent metabolic event 702 |
| ▩ Insulin regimen nonadherent metabolic event 704 |

Fig. 10

Fig. 11

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012152295 A2
- WO 2014037365 A1
- WO 2010149388 A2
- WO 2010149388 A

**Non-patent literature cited in the description**

- **SCHMID.** New options in insulin therapy. *J Pediatria (Rio J),* 2007, vol. 83 (5), S146-S155
- **PLANK et al.** A double-blind, randomized, dose-response study investigating the pharmacodynamic and pharmacokinetic properties of the long-acting insulin analog detemir. *Diabetes Care,* 2005, vol. 28, 1107-1112
- **HELMS KELLEY.** Insulin glulisine: an evaluation of its pharmacodynamic properties and clinical application. *Ann Pharmacother,* 2009, vol. 43, 658-668
- **GERICH.** Novel insulins: expanding options in diabetes management. *Am J Med.,* 2002, vol. 113, 308-316
- **DASSAU et al.** Detection of a Meal Using Continuous Glucose Monitoring. *Diabetes Care,* 2008, vol. 31, 295-300
- **CAMERON et al.** Probabilistic Evolving Meal Detection and Estimation of Meal Total Glucose Appearance. *Journal of Diabetes Science and Technology,* 2009, vol. 3 (5), 1022-1030